(19) 

Europäisches Patentamt
European Patent Office
Office européen des brevets

(11) **EP 1 955 724 B1**

(12) **EUROPEAN PATENT SPECIFICATION**

(45) Date of publication and mention of the grant of the patent:
**05.12.2012 Bulletin 2012/49**

(51) Int Cl.:
*A61M 25/00* (2006.01)   *A61B 8/12* (2006.01)

(21) Application number: **08002209.8**

(22) Date of filing: **06.02.2008**

(54) **Medical catheter**

Medizinischer Katheter

Cathéter médical

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **06.02.2007 JP 2007027272**

(43) Date of publication of application:
**13.08.2008 Bulletin 2008/33**

(73) Proprietor: **Terumo Kabushiki Kaisha Tokyo (JP)**

(72) Inventors:
• **Tanioka, Hiromichi**
**c/o Terumo Kabushiki Kaisha**
**Ashigarakami-gun**
**Kanagawa 259-0151 (JP)**

• **Yabe, Kouji**
**c/o Terumo Kabushiki Kaisha**
**Ashigarakami-gun**
**Kanagawa 259-0151 (JP)**

(74) Representative: **Casalonga, Axel et al**
**Casalonga & Partners**
**Bayerstrasse 73**
**80335 München (DE)**

(56) References cited:
EP-A- 0 841 072       WO-A-00/13733
WO-A-2004/033015       WO-A-2005/018727
US-A- 5 676 659       US-A- 5 695 483

**Description**

TECHNOLOGICAL FIELD

The present invention generally relates to a medical catheter intended for diagnosis and therapy in a body cavity. More particularly, the invention pertains to a medical catheter falling under the category of a high-functioning catheter which permits insertion of diagnostic and therapeutic devices into the body cavity while exhibiting good kink resistance.

BACKGROUND DISCUSSION

One common method for diagnosis and therapy involves inserting a high-functioning catheter into the body cavity. These types of catheters include, for example, vessel expanding catheters adapted to expand an arteriosclerotic stenotic lesion, vessel expanding catheters for stent indwelling, catheters for ultrasonic diagnosis, and catheters for optical coherence tomography.

Medical catheters characterized as high-functioning catheters are usually required to be small in diameter with a small (thin) wall thickness. In addition, the vessel expanding catheter as a typical example of a high-functioning catheters is required to be able to reach a peripheral vessel through a fine meandering branched vessel. It should be composed of a distal portion which is flexible and a proximal portion which permits easy torque transmission and easy pushing without kinking.

The catheter for intravascular ultrasonic diagnosis, which permits a diagnostic device to pass through it for diagnostic imaging, should be composed of a distal portion which is flexible and highly transparent to ultrasonic waves and a proximal portion which permits easy torque transmission and easy pushing without kinking.

The catheter for optical coherence tomography should also be composed of a distal portion which is made of a transparent flexible resin material and a proximal portion which permits easy torque transmission and easy pushing without kinking.

As mentioned, high-functioning catheters to be used as medical catheters should be small in diameter and small (thin) in wall thickness. In addition, they should be composed of a relatively flexible distal portion and a relatively stiff proximal portion. Ideally, they should be constructed such that they gradually change in stiffness in their lengthwise direction.

The catheter will be relatively poor in performance if it does not have a gradual change of stiffness, but rather has an uneven distribution of stiffness. That part of the catheter where stiffness changes abruptly tends to cause kinking due to concentrated stress.

A high-functioning catheter proposed so far that is intended to meet the above-mentioned requirements is constructed as follows. The proximal portion is a tubular structure composed of regularly braided metal wires and resin layers covering both sides of the braid. The tubular structure is very stiff and has a small diameter and a thin wall thickness. In addition, the proximal portion gradually decreases in stiffness from its proximal end to its junction with the distal portion.

Among the known medical catheters constructed as mentioned above is one which is disclosed in U.S. Patent No. 5,533,987 (hereinafter referred to as Patent Document 1). The disclosed catheter is a vessel expanding catheter composed of a proximal portion made of polyimide resin and a distal portion in the form of resin tube.

The proximal portion is a resin-coated tube braided from stainless steel wires. In addition, the proximal portion is constructed such that the braid becomes denser and the flexibility increases extending to the junction between the proximal portion and the distal portion.

Another type of medical catheter is disclosed in Japanese Patent Laid-open No. 2001-178826 (hereirtafter referred to as Patent Document 2). This catheter is composed of an inner tube, a braided layer, and an outer tube, with the inner and outer tubes being fused together. It is so constructed as to gradually decrease in stiffness in extending to the distal portion. This structure results from the tube decreasing in stiffness and the braid decreasing in density in extending to the distal portion.

In the case of a high-functioning catheter, the distal portion is usually a resin tube which needs flexibility. This is not true for the catheter disclosed in Patent Document 1, in which the proximal portion is a braided tube with a coating. The braid is formed from stainless steel wires and the coating (at least one layer thereof) is formed from a hard polyimide resin.

Therefore, the proximal portion constructed in this manner is much stiffer than the resin tube constituting the distal portion, even though the braided layer is made flexible by increasing the mesh density. The problem encountered in this case is that stress concentration is liable to occur at the junction between the proximal portion (or the braided tube) and the distal portion (or the resin tube), which leads to kinking.

There may be a conceivable way of imparting gradually increasing flexibility to the braided tube by using wires which gradually decrease in diameter for the braid layer.

The braid layer of wires should usually be formed on a core wire coated with a material that becomes the inner layer of the tube. In other words, the braid layer should be formed on a coated core wire. The tubular braid layer is formed by using a special apparatus called a braider. The braider is provided with bobbins on which thin wires are wound, and each bobbin is provided with a spring to adjust the tension of the wire. The spring should have an adequate spring constant according to the wire size so that the wires are braided into a desirable braid layer.

Any spring with an excessively small spring constant results in a small wire tension, an irregular mesh density, and entanglement. By contrast, any spring with an excessively large spring constant results in a large wire tension, which causes the wire to cut into the inner layer of the tube, causing damage.

It is desirable that the braided layer be formed from wires having a constant wire size. However, forming the braided layer from wires with a gradually decreasing diameter is not practicable because tension control is difficult for the reasons mentioned above.

The catheter for ultrasonic diagnosis should have a cavity to hold a diagnostic device therein for imaging. Therefore, its distal portion should be made of a material, such as resin, which is not only transparent to ultrasonic waves but also flexile, so that it functions as a window for observation.

The catheter for optical coherence tomography should also have a cavity to hold a diagnostic device therein for imaging. Therefore, its distal portion should be made of a material, such as resin, which is not only transparent to light but also flexile, so that it functions as a window for observation.

In any case, problems arise at the junction between the proximal portion, which is a tube having a braided layer, and the distal portion, which is a tube made of flexible resin. The high-functioning catheter requires that the junction between the proximal portion and the distal portion has the smallest step difference between the inner and outer surfaces of the tube and that the junction causes no kinking and has a sufficient tensile strength at break. Unfortunatety, those catheters disclosed in Patent Documents 1 and 2 mentioned above do not meet these requirements.

Other types of medical catheters are disclosed in documents WO 2005/018727, WO 00/13733 and EP 0 841 072.

## SUMMARY

The medical catheter disclosed here provides an improved medical catheter relative to other known catheters. The medical catheter here is suitable for use as a high-functioning catheter and is composed of a proximal portion and a distal portion, the proximal portion being a resin tube of composite material having a gradually decreasing high stiffness, and the proximal portion and the distal portion being connected together in such a way as to improve kinking resistance.

One aspect involves a medical catheter comprising a distal tube portion, and a proximal tube portion, wherein the proximal tube portion includes a metal layer extending longitudinally within the proximal tube portion, and at least a part of the metal layer being subjected to a cross-sectional area reducing treatment resulting in the cross-sectional area of the metal layer varying along the longitudinal extent of the metal layer. The medical catheter includes a tubular distal part and a tubular proximal part, with the latter preferably being a multilayered structure (or composite body) composed of a metal layer and a resin layer.

More specifically, the proximal tube portion is made as a tube including a metal mesh, and is a multilayered tube composed of an inner layer, a metal layer (or metal mesh layer in this case), a coating layer (which fixes the metal layer to the inner layer), and an outer layer, which are sequentially formed one over another.

The medical catheter may be prepared by combining together the distal tube portion and the proximal tube portion which have been prepared separately, or by preparing the distal tube portion and the proximal tube portion integrally all at once.

In any case, the metal layer gradually changes in stiffness because it has a cross-sectional area which varies in the lengthwise direction of the proximal tube portion. The gradually changing stiffness is realized by forming the metal layer from flat wires and by performing physical or chemical polishing on the metal layer. The chemical polishing is etching on a specific part at which stiffness should gradually change.

The coating layer fixes the metal layer to the inner layer after the former has been formed on the latter. The coating layer is covered with the outer layer which imparts stiffness as desired and also functions as a protective layer.

The coating layer should be formed in such a way that it gradually decreases in thickness so that the proximal tube portion becomes more flexible in going from its proximal to its distal end. Adjusting the thickness in this way imparts gradually changing stiffness to the proximal tube portion.

Both or either of the inner layer and the outer layer should have gradually changing stiffness. This object is achieved by combining together a plurality of materials differing in stiffness or by using materials differing in thickness.

The proximal tube portion and the distal tube portion are connected together to form the medical catheter. The boundary between the two components constitutes the junction. The junction is formed in such a way that the resin tube has a minimum step and a minimum difference in stiffness between the inner layer and the outer layer. For example, the distal tube portion should have a flaring terminal opening for connection to the proximal tube portion and the proximal tube portion should have a tapered terminal opening for connection to the distal portion. The tapered terminal opening is formed by peeling off the outer layer over a certain length so that the coating layer is exposed. Connection of the distal tube portion and the proximal tube portion at the junction is accomplished by fusing or bonding.

In the case of an integral-type medical catheter in which a single tube passes through the distal tube portion and the proximal tube portion, the entire tube is constructed of the inner layer and the outer layer and only the proximal tube portion is provided with a metal layer and a coating layer to fix it between the inner layer and the outer layer. The neighborhood of the end of the metal layer and coating layer (adjacent to the distal tube portion) functions as the boundary.

Therefore, the integral-type medical catheter may be prepared by forming the inner layer over the entire length of the tube, forming the metal layer and coating layer on the proximal tube portion only, and finally forming the outer layer over the entire length of the tube.

The medical catheter constructed as mentioned above functions satisfactorily because of its structure as well as its selected materials. It will find use as high-functioning catheters such as vessel expanding catheters to expand the arteriosclerotic stenotic lesion, vessel expanding catheters for stent indwelling, catheters for ultrasonic diagnosis, and catheters for optical coherence tomography.

The medical catheter here is preferably composed of a distal tube portion and a proximal tube portion, the former having more than one resin layer and the latter having a metal layer and more than one resin layer. The metal layer in the proximal tube portion is physically or chemically polished and varies in cross-sectional area in its lengthwise direction.

The proximal tube portion with a metal layer has gradually changing stiffness as desired.

One advantage of the above-mentioned structure is that the proximal tube portion has a lower stiffness at its distal end than at its proximal end, and this imparts good kink resistance to the boundary (or junction) between the distal tube portion and the proximal tube portion. Moreover, such gradually changing stiffness makes the distal end of the proximal portion flexible as desired and improves the proximal tube portion's torque transmission and pushing ability, which is required of high-functioning catheters.

Another aspect involves a method for producing a medical catheter comprised of a distal tube portion and a proximal tube portion. The method comprises positioning metal wires forming a metal layer around an inner layer of the proximal tube portion so that the metal layer extends longitudinally along the proximal tube portion, and treating a distal portion of the metal layer to reduce a cross-sectional area of a portion of the longitudinally extending metal layer so that the cross-sectional area of the metal layer varies along a longitudinal extent of the metal layer.

The treatment of the distal portion can involve physical or chemical polishing. This makes the metal layer of the proximal tube portion vary in cross-sectional area from one place to another and also makes the proximal tube portion less stiff at its distal end. This structure imparts good kink resistance to the boundary between the proximal tube portion and the distal tube portion.

The metal layer may be formed from flat wires, and the resulting metal mesh layer may undergo physical or chemical polishing. In this case, the metal layer is easily given gradually changing stiffness.

The metal mesh layer is embedded between the inner tube and the coating layer formed thereon. This structure surely imparts gradually changing stiffness to the medical catheter.

BRIEF DESCRIPTION OF THE DRAWING FIGURES

Fig. 1 is a plan view of a medical catheter disclosed herein which is used as a catheter for ultrasonic diagnosis.

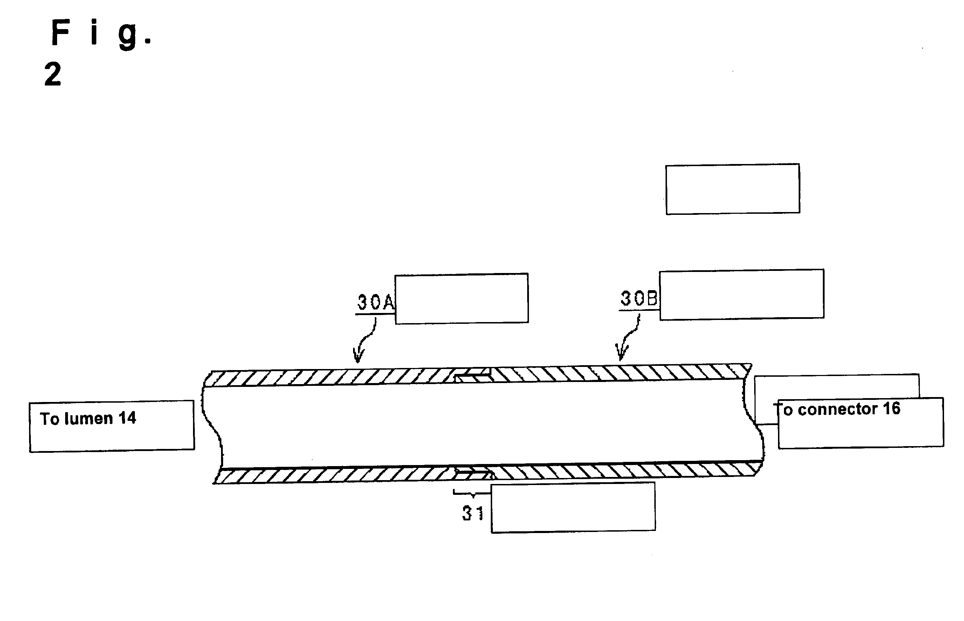

Fig. 2 is a longitudinal cross-sectional view of one portion of the medical tube shown in Fig. 1.

Fig. 3 is an enlarged longitudinal cross-sectional view of a portion of the catheter shown in Fig. 2.

Figs. 4A-4D are cross-sectional views of parts of the catheter shown in Fig. 3.

Figs. 5A-5C are somewhat schematic illustrations of aspects of the method for producing the medical catheter.

Figs. 6A-6C are somewhat schematic illustrations of other aspects of the method for producing the medical catheter.

Fig. 7 is a graph showing the flexural strength of parts of the medical catheter.

Fig. 8 is a graph showing the relationship between the outside diameter and the duration of etching in the case where the outer tube is formed from high-density polyethylene (HDPE).

Fig. 9 is a graph showing the relationship between the outside diameter and the duration of etching in the case where the outer tube is formed from polybutylene terephthalate (PBT).

Fig. 10 is a graph showing the relationship between the outside diameter and the duration of etching in the case where the outer tube is formed from polyether ether ketone (PEEK).

Fig. 11 is a graph showing the relationship between the flexural strength and the duration of etching in the case where the outer tube is formed from high-density polyethylene (HDPE).

Fig. 12 is a graph showing the relationship between the flexural strength and the duration of etching in the case where the outer tube is formed from polybutylene terephthalate (PBT).

Fig. 13 is a graph showing the relationship between the flexural strength and the duration of etching in the case where the outer tube is formed from polyether ether ketone (PEEK).

Fig. 14 is a graph showing how the flexural strength changes in the lengthwise direction over the entire length (from the proximal end to the distal end) of the medical tube.

Fig. 15 is a diagram showing parts of a medical catheter according to another embodiment.

Figs. 16A-16C are somewhat schematic illustrations of aspects of the method for producing the medical tube shown in Fig. 15.

Figs. 17A-17C are somewhat schematic illustrations of other aspects of the method for producing the medical catheter.

DETAILED DESCRIPTION

The aspects of the medical catheter described below are applicable to medical catheters of the connection type which are composed of a distal tube portion and a proximal tube portion joined together after being prepared separately, and are also applicable to medical catheters of the one-piece type which are composed of a distal tube portion and a proximal tube portion formed integrally all at once and at the same time.

The medical catheter according to embodiments described below is usefully applied to high-functioning catheters such as, for example, catheters for ultrasonic diagnosis, catheters for vessel expansion, and catheters for stent indwelling.

The following is a detailed description of the medical catheter of the connection type according to one embodiment disclosed herein.

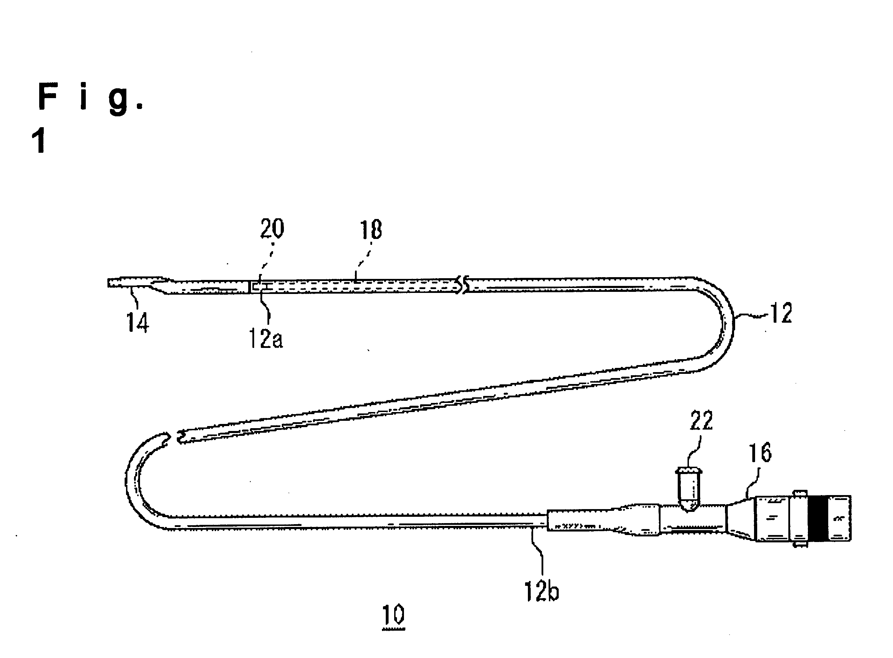

Fig. 1 illustrates a catheter 10 as a high-functioning catheter for ultrasonic diagnosis. The catheter 10 includes a medical tube 12 forming a catheter sheath. The medical tube 12 can include a short tube 14 having a lumen (lumen-containing short tube) for receiving the guide wire at its distal end 12a and also has the connector 16 at its proximal end 12b.

The medical tube 12 has an inside diameter large enough for a drive shaft 18 to pass through. The drive shaft 18 has an ultrasonic transducer 20 at its distal end.

The connector 16 is provided with an external drive source (not shown) which drives the drive shaft 18 so that the ultrasonic transducer 20 turns through 360°. This structure permits scanning (for diagnostic imaging) around the blood vessel.

The connector 16 also has an injection port 22 for priming. The injection port 22 is used to supply the medical tube 12 with a liquid.

The medical tube 12 mentioned above is composed of a distal tube or distal tubular portion 30A and a proximal tube or proximal tubular portion 30B. These two tubes are joined together at a junction 31 so that they together form an integral body.

The distal tube 30A is a single-structure body formed from of a resin layer (which is referred to as a resin tube hereinafter). In other words, the distal tube 30A is formed of only one material, the resin or single resin layer. On the other hand, the proximal tube 30B is a multilayered body composed of a metal layer and resin layers. (The proximal tube 30B may occasionally be referred to as a tube with a metal mesh because the metal layer is preferably a layer of a metal mesh.)

The medical tube 12, when used as a catheter for diagnosis and therapy in a blood vessel, should preferably have an overall length of about 1000 to 1500 mm. It should also have an outside diameter smaller than 1.5 mm, preferably smaller than 1 mm, so that it can be relatively easily inserted into a blood vessel. In addition, it should have a wall thickness smaller than 0.2 mm, particularly smaller than 0.1 1 mm, so that it has adequate flexibility as well as adequate stiffness. The distal tube 30A should preferably have an approximate length of 100 to 300 mm.

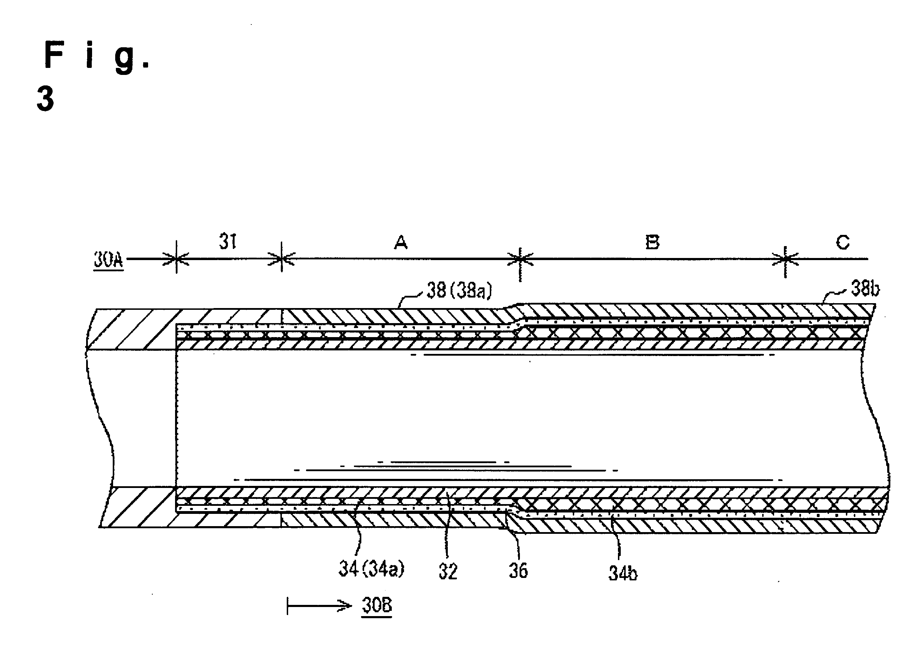

The proximal tube 30B as a constituent or part of the medical tube 12 is of multilayered structure. The structure of the proximal tube 30B will be described below in more detail with reference to Fig. 3 which is an enlarged cross-sectional view. In the description below, "distal" denotes the direction to the end adjacent to the distal tube 30A, and "proximal" denotes the direction to the end adjacent the connector 16.

As shown in Fig. 3, the proximal tube 30B is composed of an inner tube 32, a metal layer 34 formed on and encircling (surrounding) the inner tube 32, a coating layer 36 fixing the metal layer 34 to the inner tube 32, and an outer tube 38 formed on and encircling (surrounding) the coating layer 36.

The metal layer 34 is a metal mesh layer. For the sake of convenience, the metal mesh layer 34a closer to or at the distal end (or adjacent to the distal tube 30A) is called the metal mesh layer closer to the junction, and the metal mesh layer 34b closer to or at the proximal end is called the metal mesh layer closer to the proximal side. A similar nomenclature is applied to the outer tube 38. That is, the outer tube 38a closer to or adjacent to the distal tube 30A is called the outer tube closer to the junction, and the outer tube 38b closer to or at the proximal end is called the outer tube close to the proximal side.

The proximal tube 30B includes three segments A, B, and C as shown in Fig. 3, with the segment A being closer to the junction 31 and the segment C being closer to the proximal end 12b. The metal mesh layer 34a closer to the junction extends over, and is embedded in, the entire length of the junction 31 and the segment A. The metal mesh 34b closer to the proximal end extends over, and is embedded in, the entire length of the segments B and C.

The whole of the distal tube 30A and the segment A of the proximal tube 30B constitute that part of the medical tube which preferably needs high flexibility. This is because the distal tube 30A and the segment A approach the vicinity of the aortic arch when the high-functioning catheter is advanced into the blood vessel. These sections should permit the distal end of the catheter to advance relatively smoothly along the aortic arch which curves sharply.

Therefore, if the distal tube 30A is about 200 mm long, the length of the junction 31 plus the length of the segment A should be about 100 to 400 mm, particularly about 200 mm.

The outer tube 38 is also given adequate flexibility for the same reason as above. That is, the outer tube 38 varies in thickness in going from one section (corresponding to the junction 31 and the segments A and B) to the proximal section. Alternatively, the outer tube 38 can be made of materials differing in stiffness to impart the desired adequate flexibility.

In this example, resin tubes differing in stiffness are used. In the section of the outer tube extending from the distal end of the junction 31 to the proximal end of the segment B, including segment A, the outer tube 38a closer to the junction is a relatively flexible resin tube. In the section of the outer tube covering the segment C down to the proximal end 12b, the outer tube 38b closer to the proximal side is a relatively stiff resin tube (e.g., is more stiff than the section of the outer tube extending over the junction 31, the segment A and the segment B).

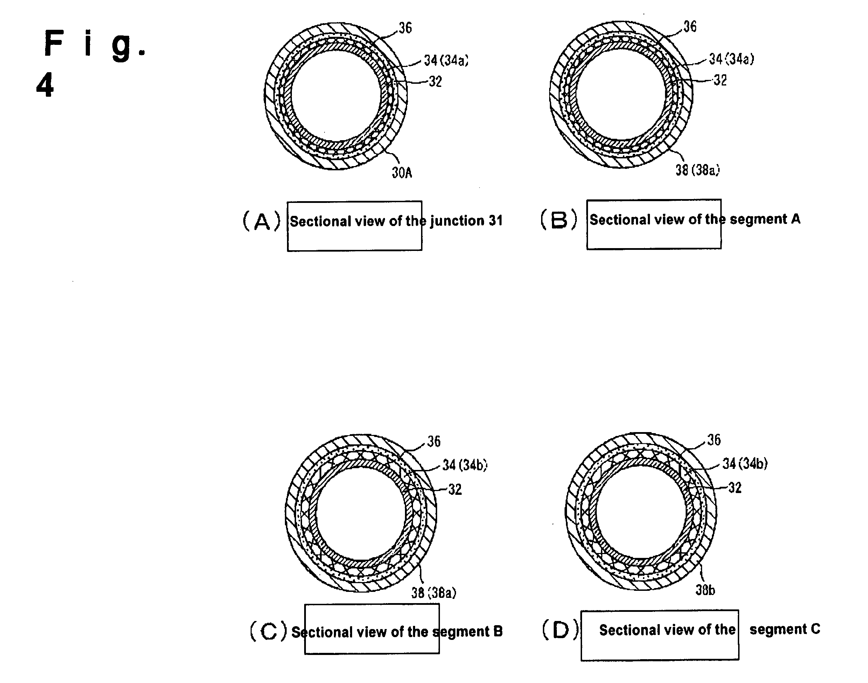

Figs. 4(A)-(D) are cross-sectional views of the medical catheter shown in Fig,. 3 at different positions as shown in Fig. 3. Fig. 4A is a cross-sectional view at the junction 31, Fig. 4B is a cross-sectional view at the segment A, Fig. 4C is a cross-sectional view at the segment B, and Fig. 4D is a cross-sectional view at the segment C.

The parts or segments of the medical catheter, including those at which the cross-sectional views in Figs. 4 (A)-(D) are taken, are described below one by one.

(1) Distal tube 30A

The distal tube 30A is constructed of a resin tube alone. Since the distal tube 30A preferably requires flexibility, the resin tube is a flexible resin tube. The catheter 10 for ultrasonic diagnosis is designed such that the ultrasonic transducer 20 (or a device for diagnostic imaging) is inserted into the resin tube for diagnosis. Therefore, the distal portion of the catheter should be relatively flexible as well as transparent to ultrasonic waves (so that it functions as a window for observation). The distal tube is formed from any resin that meets the foregoing requirement. A discussion of additional aspects of the distal tube is set forth below.

The catheter for optical coherence tomography into which a device for diagnosis is inserted also needs a transparent flexible resin tube. Transparency is necessary for the resin tube to function as a window for observation.

(2) Junction 31

The junction 31 is a part at which the distal tube 30A and the proximal tube 30B are joined together. This junction 31 should have sufficient tensile strength and a minimum variation in its inside and outside diameters. Also, the junction 31 should be such that a minimum difference in stiffness exists between the distal tube 30A and the segment A.

These requirements are met when the metal mesh layer 34a closer to the junction is formed from thin flat metal wires. In addition, the coating layer 36 on this metal mesh layer should ideally be thinner than that closer to the proximal end 12b. For good connection, the inner tube 32 should preferably be formed from the same material (resin tube) as used for the distal tube 30A.

(3) Segment A

The segment A constitutes the distal end of the proximal tube 30B. In terms of stiffness of the proximal tube 30B (i.e., the relative stiffness of segments A-C), segment A is the most flexible, segment B is more stiff than segment A, and segment C is more stiff than segment B (i.e., segment C is more stiff than both segment A and segment B). Also, the junction 31 is preferably more flexible than segment A. The flexibility/stiffness of segment A is preferably the same throughout its length.

This requirement is met when the metal mesh layer (of metal wires) as the metal layer 34a closer to the junction is thin, the coating layer 36 is also thin, and the outer tube 38a closer to the junction is formed from a resin material as stiff as the distal tube 30A or stiffer than the inner tube 32 of the junction 31.

(4) Segment B

The segment B constitutes approximately the middle part of the proximal tube 30B. As noted above, this segment B should be more stiff than segment A, but not as stiff as segment C. The flexibility/stiffness of segment B is preferably the same throughout its length. This requirement is met when the metal mesh layer 34b closer to the junction in the segment B is thicker than the metal mesh layer 34a in the segment A. Likewise, the coating layer 36 should be thicker in the segment B than in the segment A.

Moreover, the outer tube 38a closer to the junction should be as stiff as or stiffer than the resin tube of the segment A.

(5) Segment C

The segment C is that part of the proximal tube 30B (having a metal mesh) which is closer to the proximal end 12b. As noted above, this segment C is the stiffest of the three segments A, B, C. This greater stiffness is desired or required for the catheter to reach a peripheral vessel,. This segment C also needs good kink resistance.

Therefore, the metal mesh layer 34b closer to the proximal end should be formed from a metal wire which is thicker than that used for the metal mesh layer 34a closer to the junction. In addition, the coating layer 36 in the segment C should be as thick as or thicker than that in the segment B. Additionally, the outer tube 38b closer to the proximal end should be a resin tube which is stiffer than that constituting the segment A or segment B. That is, the outer tube 38 in the segment C is stiffer than the outer tube in the segments A and B.

The following describes materials most preferably suitable for each constituent or part of the medical catheter.

(a) Distal tube 30A

The distal tube 30A is a resin tube which needs to reach the meandering peripheral vessel. Therefore, the resin tube should be not only flexible, but also transparent to ultrasonic waves so that it functions as a window for observation.

The resin tube should also have a precisely finished inside diameter and outside diameter so that it permits the passage of a device for diagnosis or therapy.

The high-precision resin tube meeting the foregoing requirements is preferably formed by using a high-precision extrusion molding machine, which accurately extrudes a molten resin through a specially designed die onto a core wire (copper wire).

The extrusion molding should employ a thermoplastic resin material, preferably one which is flexible and transparent.

These requirements are met by resins such as high-density polyethylene, low-density polyethylene, linear low-density polyethylene, and polyethylene elastomer.

The foregoing polymer materials are highly transparent to light and ultrasonic waves, and hence are suitable for those medical catheters which emit light or ultrasonic waves.

The foregoing resins may be used in the form of a polymer alloy or a polymer blend. In addition, the distal tube 30A may be a multilayered or striped resin tube composed of several kinds of resin.

When the medical catheter is used as a high-functioning catheter, the distal tube 30A should preferably have a length and diameter as specified above.

(b) Proximal tube 30B

(b1) Inner tube 32

The inner tube 32 should be formed on a core wire (copper wire) because it is covered with a mesh layer of metal wires. Therefore, it should be formed from a thermoplastic polymeric material that can be extruded in a relatively thin layer onto a core wire, or from a soft or hard resin material having a desirable stiffness according to use.

With respect to the thermoplastic polymeric materials, preferred hard resin materials include polyolefin resins such as high-density polyethylene, polypropylene, polybutene, polyvinyl chloride, and ethylene-vinyl acetate copolymer, polyolefin elastomers, fluororesin or fluoroelastomer, methacrylic resin, polyphenylene oxide, modified polyphenylene ether, polyethylene terephthalate, polybutylene terephthalate, polyether ether ketone, polyamideimide, polyetherimide, polyethersulfone, cyclicpolyolefin, polyurethane elastomer, polyester elastomer, polyamide or polyamide elastomer, polycarbonate, polyacetal, styrene resin or elastomer, and thermoplastic polyimide.

These resin materials may be used in the form of a polymer alloy or polymer blend. Also, these resin materials may be used in combination to form a multilayered resin tube or a striped resin tube.

Of the thermoplastic polymeric materials, preferred soft materials include polyolefin resins such as high-density polyethylene, low-density polyethylene, linear low-density polyethylene, polyethylene elastomer, polypropylene elastomer, polybutene elastomer, soft polyvinyl chloride, and ethylene-vinyl acetate copolymer and thermoplastic elastomers such as polyolefin elastomer, fluoroelastomer, polyurethane elastomer, polyester elastomer, polyamide elastomer, and styrene elastomer.

These resin materials may be used in the form of a polymer alloy or polymer blend. In addition, these resin materials may be used in combination to form a multilayered resin tube or a striped resin tube.

The inner tube 32 may be a resin tube having a uniform stiffness distribution (i.e., the stiffness of the inner tube 32 is the same along the length of the inner tube 32) or varying in stiffness from one place to another along its length. In the latter case, the inner tube 32 may be formed from a plurality of resin tubes differing in stiffness, so that the stiffness decreases from the part closer to the proximal end 12b to the part closer to the distal tube 30A.

The resin tube having a gradually changing stiffness can be obtained by forming a plurality of resin tubes of the same material, but differing in stiffness, on a core wire and subsequently connecting them together by fusion bonding.

Several resin materials may vary in stiffness as desired if they have the same or similar chemical structure but vary in molecular weight. Their stiffness depends on the ratio of the soft segment and the hard segment or the ratio of the soft resin material and the hard resin material.

To be more specific, the hard resin material may be polyamide 12 and the soft resin material may be polyamide elastomer. Alternatively, the hard resin material may be polybutylene terephthalate and the soft resin material may be polyester elastomer.

The inner tube 32 composed of several longitudinally arranged sections varying in stiffness may be formed by a method involving forming resin tubes varying in stiffness (each having a slightly larger inside diameter than the diameter of a core wire on which they are slipped on later), slipping the sections on a core wire, covering the resin tubes with a heat-shrinkable tube, heating the entire assembly, and finally removing the core wire. In this way there is obtained the inner tube which gradually changes in stiffness in the lengthwise direction.

It is also possible to control stiffness by changing the wall thickness of the inner tube 32. In this case, the wall thickness close to the proximal end is made thicker by, for example, coating a core wire which varies in diameter.

The inner tube 32 may also be formed by extrusion of several resin materials varying in stiffness in such a way that the resin materials are switched from a hard one to a soft one as the extrusion proceeds to coat a core wire with the resin materials. This extrusion method may be combined with the foregoing method which makes that part closer to the proximal end 12b thicker owing to a core wire which gradually changes in diameter. The resulting inner tube 32 will have a much stiffer part closer to the proximal end 12b.

The inner tube 32 may be one which is formed from a single resin material on a core wire, with the same stiffness throughout the length of the inner tube 32. In this case, it is uniform in stiffness over the entire length.

The inner tube 32, which is continuously formed on a core wire by the above-mentioned method, permits the metal wire mesh (metal braid) to be formed thereon continuously and stably by a braider. This leads to greatly improved productivity and reliability.

The inner tube 32 may be formed by any method other than those mentioned above. For example, it may be formed from a thermosetting resin by coating on a core wire and subsequent heating for curing.

The thermosetting resin suitable for this purpose includes thermosetting polyimide resin, urethane resin, epoxy resin, and silicone resin.

Coating with one of these thermosetting resins may be accomplished by any of the following three methods. A first method involves dissolving the thermosetting resin in a solvent to give a solution with an adequate viscosity, dipping a core wire in the solution, and heating the coated core wire in a heating furnace for a prescribed period of time at a temperature higher than the curing temperature.

In this case, coating may be repeated more times at one part than another. In this way, it is possible to change the thickness of coating in the lengthwise direction of the tube. Thus there is obtained an inner tube which gradually changes in stiffness due to thickness variation.

A second method involves mixing polytetrafluoroethylene powder with an emulsifier for uniform dispersion, dipping a core wire in the emulsion for coating, and heating at a temperature above the melting point of polytetrafluoroethylene.

A third method involves coating a core wire with a photopolymerizable material, followed by irradiation with light for curing.

The photopolymerizable materials include, for example, ultraviolet-curable resins and visible light-curable resins. Any one of these resins is dissolved in a solvent to give a solution with an adequate viscosity, and a core wire is dipped in this solution for coating. The resin coating is cured by irradiation with UV rays or visible light.

Coating may be repeated more times at one part than another. Thus the thickness of coating can be changed, and there is obtained an inner tube 32 which gradually changes in stiffness.

Incidentally, in the case of a medical catheter for use as a high-functioning catheter, the inner tube 32 should have an outside diameter smaller than 1.5 mm, preferably smaller than 1 mm, and a thickness smaller than 0.1 mm, particularly smaller than 0.05 mm.

(b2) Metal layer (metal mesh layer) 34

The inner tube 32 formed as mentioned above is subsequently covered with the metal layer 34. In this illustration, the metal layer is a metal mesh layer. The metal mesh layer may be formed from a plurality of thin fine flat metal wires. The metal wires may be wires of stainless steel, titanium, tungsten, iron, or boron.

As mentioned previously, the metal mesh layer may be formed by using a specially designed apparatus called a braider. The braider has a plurality of parts called carriers, each of which holds a bobbin having a flat wire wound thereon. The carriers are regularly moved in the mutually opposite direction to achieve braiding. In this way the tubular metal mesh layer is braided with metal wires crossing one another.

The carriers are provided with a spring for adjustment of wire tension. In order to form a good metal mesh layer, it is necessary to select an adequate spring constant according to the wire size.

As mentioned above, any spring with an excessively small spring constant may result in a small wire tension, an irregular mesh density, and entanglement. By contrast, a spring with an excessively large spring constant may result in a large wire tension, which causes the wire to cut into the inner tube 32, causing damage to the inner tube 32.

The metal mesh layer 34 should preferably be formed from a flat metal wire with a uniform thickness, which permits easy tension control. Such a metal wire gives the metal mesh layer 34 a generally consistent braiding without wires being displaced.

The flat wire for the metal mesh layer 34 should have a width of 0.05 to 0.15 mm, preferably 0.06 to 0.1 mm, and a thickness of 0.01 to 0.1 mm, preferably 0.01 to 0.05 mm.

The metal mesh layer 34 possesses a mesh density which is uniform or variable over the length from the proximal end 12b to the junction 31.

The metal mesh layer 34 should preferably vary in stiffness in the lengthwise direction of the tube. The gradually changing stiffness is preferably obtained by changing or varying the cross-sectional area of the flat metal wire constituting the metal mesh. For the tube to vary in flexibility in its lengthwise direction, it is preferable to use a flat wire which becomes thinner toward the junction 31. That is, the cross-sectional area of the metal mesh layer 34 varies or becomes smaller towards the junction. Thus, the cross-sectional area of the metal mesh wire 34 at location along the metal mesh wire closer to the junction 31 is smaller than at a location closer toward the proximal end 12b.

The flat metal wire may have its cross-sectional area changed or varied in an appropriate manner through use of a cross-sectional area reducing treatment, for example by physical or chemical polishing or treatment.

Methods for physical polishing include grinding, blasting, barrel tumbling, and liquid honing. These methods can be accomplished by bringing the metal mesh layer 34 into direct contact with a grinding wheel or abrasive grains.

The cross-sectional area (or thickness) of the wire can be optimized by controlling the duration of polishing. Adjusting the duration of polishing at a specific part of the wire gradually changes the cross-sectional area in the lengthwise direction of the wire. In this way the stiffness of the tube changes gradually in the lengthwise direction.

A typical method for chemical polishing involves etching with a chemical (etchant) that dissolves the flat metal wire. Etching solutions for SUS304 flat wire include, for example, aqueous solutions of sulfuric acid, hydrochloric acid, and ferric chloride.

Etching can be carried out in the following way. The inner tube 32 on which the metal mesh layer 34 has been formed has both of its ends sealed with an adhesive so that the braided wires of the metal mesh layer 34 are fixed. Then, the inner tube 32 having the metal mesh layer 34 thereon is dipped in the etching solution. After a certain period of time, the inner tube 32 having the metal mesh layer 34 thereon is removed from the etching solution and washed with water. Etching may optionally be followed by such post treatment as neutralization and passivation.

Since the material forming the metal mesh layer 34 is subjected to chemical polishing with an etching solution, the part of the metal mesh layer 34 on which etching is carried out becomes thinner than the other part. The degree of thinning depends on the duration of etching.

The duration of etching may be adjusted by removing the inner tube 32 having the metal mesh layer 34 thereon from the etching solution intermittently for certain lengths and/or at certain time intervals. After etching in this way, the flat wires change in cross-sectional area in a stepwise manner in the lengthwise direction. Thus the metal mesh layer 34a (or the inner tube 32 having the metal mesh layer 34) has a stiffness which gradually changes in its lengthwise direction.

(b3) Coating layer 36

The metal mesh layer 34 formed on the inner tube 32 may not be stable if it has a low mesh density. In this case, the flat wires constituting the metal mesh layer 34 might be subject to dislocation, thus causing variations in mesh density.

If the metal mesh layer 34 is covered with the outer tube 38 while the flat wires are not yet fixed to the outer surface of the inner tube 32, the outer tube 38 is liable to kink near the proximal end 12b and so the desired stiffness may be difficult to achieve. Moreover, the proximal tube 30B in this state has a much lower tensile break strength than necessary for the medical catheter.

Presumably, this is because the outer tube 38 does not completely enter the interstices of the metal mesh layer 34. Thus, the inner tube 32, the metal mesh layer 34, and the outer tube 38 exist separately without firm bonding.

To impart mechanical properties required of the medical catheter 10, it is preferable not only to form the metal mesh layer 34 on the outer surface of the inner tube 32, but also to fix the metal mesh layer 34 to the inner tube 32.

With the metal mesh layer 34 fixed to the inner tube 32, the proximal tube 30B (having the metal mesh) exhibits the characteristic properties of the composite material, and hence it can be used for the medical tube with desirable mechanical properties.

Fixing the metal mesh layer 34 to the inner tube 32 is preferably accomplished by coating with a thermosetting resin. This coating is carried out after the metal mesh layer 34 has been formed. The resulting coating layer 36 fills the interstices of the metal mesh layer 34 on the inner tube 32, thereby joining the three components together firmly and mechanically. The thus joined components exhibit the mechanical properties of the composite material.

Thermosetting resins to be used as the coating material include, for example, polyimide resin, epoxy resin, urethane resin, and silicone resin. Coating is preferably accomplished by dissolving a thermosetting resin in a solvent to give a solution having adequate viscosity, dipping the inner tube 32 in the solution, and heating in a heating furnace at a temperature higher than the curing temperature for a prescribed period of time. As the result of this, the coating layer 36 adheres to the inner tube 32 and the metal mesh layer 34, thereby making the three components mechanically integral.

The resin material for the inner tube 32 should withstand the heating temperature of the coating material of the coating layer. Otherwise, the inner tube 32 could deform when the coating material is heated for curing.

If the coating step(s) is repeated more times at a part near the proximal end 12b than at any other part, the resulting coating layer 36 varies in thickness in the lengthwise direction. This variation in thickness leads to a gradual change in stiffness.

The coating material includes, in addition to thermosetting resins, those photopolymerizable resins which cure upon irradiation with UV light or visible light.

Coating with a photopolymerizable resin is accomplished by dissolving it in a solvent to give a solution having an adequate viscosity, dipping the inner tube 32 in the solution, and irradiation with UV light or visible light.

(b4) Outer tube 38

The medical catheter 12 is completed by joining together the distal tube 30A and the proximal tube 30B in the junction 31. It is preferred that there exists no step between the two tubes joined together. Thus, the distal tube 30A and the proximal tube 30B have the same outside diameter. In the junction 31, the inner tube 32, the mesh layer 34 and the coating layer 36 intrude or protrude into the proximal end of the distal tube 30A, with the proximal end of the distal tube 30A overlying (lying radially outwardly of) the inner tube 32, the mesh layer 34 and the coating layer 36 as illustrated. The outer tube 38 covering the coating layer 36 has an adequate thickness so that the outer surface of the proximal tube 30B snugly fits into the outer surface of the distal tube 30A (i.e., at the junction 31) without leaving steps.

The outer tube 38 is formed from a thermoplastic resin material which imparts the desired stiffness for specific uses. The thermoplastic resin may be a soft one or a hard one.

The hard thermoplastic resins include, for example, polyolefin resins such as polyethylene, polypropylene, polybutene, polyvinyl chloride, and ethylene-vinyl acetate copolymer, polyolefin elastomers, fluororesin or fluoroelastomer, methacrylic resin, polyphenylene oxide, modified polyphenylene ether, polyethylene terephthalate, polybutylene terephthalate, polyether ether ketone, polyamideimide, polyetherimide, polyethersulfone, cyclicpolyolefin, polyurethane elastomer, polyester elastomer, polyamide or polyamide elastomer, polycarbonate, polyacetal, styrene resin or elastomer, and thermoplastic polyimide.

These resins may be used in the form of a polymer alloy or polymer blend.

The soft thermoplastic resins include polyolefin resins or elastomers such as polyethylene elastomer, polypropylene elastomer, polybutene elastomer, soft polyvinyl chloride, and ethylene-vinyl acetate copolymer, and thermoplastic elastomers such as fluoroelastomer, polyurethane elastomer, polyester elastomer, polyamide elastomer, and styrene elastomer.

These resins may be used in the form of a polymer alloy or polymer blend.

The outer tube 38 should be constructed so that it varies in stiffness from the part closer to the proximal end 12b to the part closer to the junction 31. The gradually changing stiffness can be attained by forming the outer tube 38 from longitudinally arranged resin tubes of differing stiffness.

To this end, the resin tubes are made integral on the coating layer 36 by fusion bonding.

For easy fusion bonding, the resin tubes should be formed from the same kind of resin which have the same or similar chemical structure but vary in molecular weight. Their stiffness depends on the ratio of the soft segment and the hard segment.

The hard thermoplastic resin may be selected from polyamide 12 and polybutylene terephthalate and the soft thermoplastic resin may be selected from polyamide elastomer and polyester elastomer.

The following method may be employed to make integral a plurality of resin tubes differing in stiffness. Two resin tubes (corresponding to the outer tube 38a closer to the junction and the outer tube 38b closer to the proximal end), both having a slightly larger inside diameter than the inner tube 32, are slipped on the coating layer 36. The two resin tubes are covered with a heat-shrinkable tube or the like, and the entire assembly is heated for shrinking. Heat shrinking causes the coating layer 36 and the outer tube 38 to become mechanically integral or to mechanically integrate. The heating step is followed by removal of both the core wire in the inner tube 32 and the heat-shrinkable tube.

In this way it is possible to form the proximal tube 30B (having the metal mesh) which is covered with the outer tube 38 differing in stiffness in the lengthwise direction.

(c) Surface treatment of the proximal tube 30B

As mentioned above, the proximal tube 30B should preferably have mechanical properties that are characteristic of the composite materials.

Enhanced mechanical properties may be attained by increasing the adhesion strength between the inner tube 32 and the coating layer 36, between the coating layer 36 and the metal mesh layer 34, and between the coating layer 36 and the outer tube 38. Adhesion usually depends on the wettability of materials. Good wettability needs a high surface energy.

Ways to increase the surface energy of materials include physical surface treatment such as corona discharge treatment, plasma treatment and UV light irradiation, or chemical surface treatment such as tetraetching and chromic acid treatment. An adequate method should be selected according to the object and the kind of materials. In this example, plasma treatment is employed to increase the adhesion strength, thereby imparting mechanical property characteristics to the catheter reflective of the composite materials.

(d) Junction 31 between the distal tube 30A and the proximal tube 30B

The medical catheter 12 mentioned above is composed of the distal tube 30A, which is a resin tube, and the proximal tube 30B, which is a composite tube of multilayered structure having a metal mesh.

To form the medical catheter 12 from the resin tube and the composite tube, it is necessary to join together the distal tube 30A and the proximal tube 30B. The junction 31 between the distal tube 30A and the proximal tube 30B should have as small a step, if any, as possible so that it causes no kinking. In addition, the junction should have sufficient tensile break strength. Also, it should have a sufficient margin for gluing as will become more apparent from the description below.

In this example, the foregoing results are achieved by shaping the ends for the junction as follows. The proximal end of the distal tube 30A is flared, and the distal end of the proximal tube 30B is thinned by partly removing the outer tube 38, with the coating layer 36 exposed. This exposed part functions as the gluing margin (region) of the junction 31. The flared proximal end of the distal tube 30A should be slightly larger than the outside diameter of the distal end of the proximal tube 30B and slightly longer than the gluing margin of the junction.

The easiest way of flaring the proximal end of the distal tube 30A is by heating as explained below. Flaring is accomplished by using a core wire of stainless steel slightly thicker (slightly larger in outside diameter) than the inside diameter of the distal tube 30A, with its end sharpened or tapering in a conical shape. The conical end of the core wire, which is heated above the heat distortion temperature of the resin tube, is pushed into the proximal end of the distal tube 30A. Thus the proximal end of the distal tube 30A is flared.

The end of the core wire may have a conical shape or dual taper shape. Heating may be accomplished by high-frequency induction heating or electric heating with a special mold. Any heating method may be employed that is suitable for joining. Flaring may also be accomplished by using a machine tool such as centerless grinder and disk grinder.

The gluing margin of the junction 31 should have an adequate length that ensures a sufficient tensile break strength. The adequate length is about 5 to 20 mm, preferably about 10 mm.

When the distal tube 30A and the proximal tube 30B are joined together, there should be no steps on the outer surface of the junction 31. This is achieved if the outside diameter of the proximal tube 30B has the same outside diameter as the distal tube 30A after joining.

For this reason, the junction 31 of the proximal tube 30B is stepped. The step is formed by cutting off an axially or longitudinally extending portion of the outer tube 38, thereby exposing the coating layer 36.

The two ends to be joined together undergo surface treatment which imparts a relatively high adhesion strength.

Good adhesion is obtained by increasing the surface energy of materials as mentioned above.

As mentioned above, methods for increasing the surface energy of materials include physical surface treatment such as corona discharge treatment, plasma treatment and UV light irradiation, or chemical surface treatment such as tetraetching and chromic acid treatment. Any one of them may be employed.

Joining the distal tube 30A and the proximal tube 30B may be accomplished by fusion bonding or adhesive bonding.

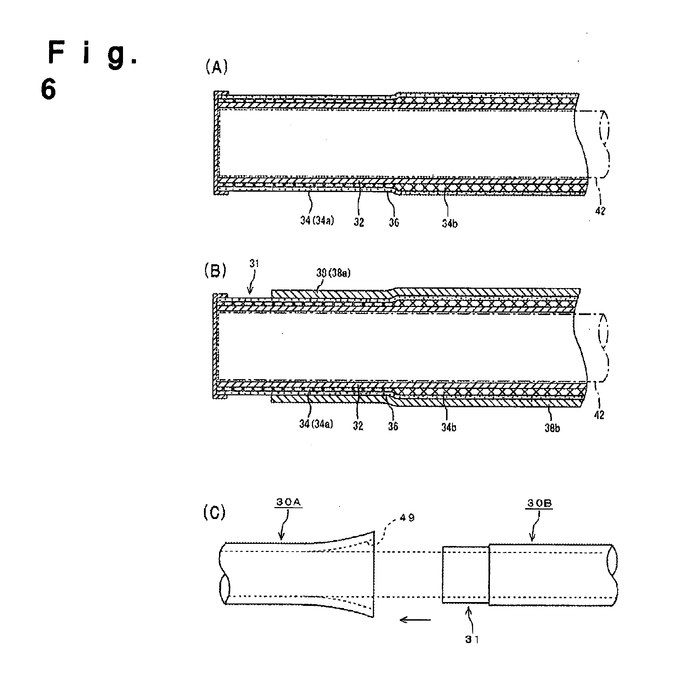

For fusion bonding by heating, the junction 31 of the proximal tube 30B is inserted into the flared proximal opening 49 of the distal tube 30A as generally illustrated in Fig. 6(C), the joint is covered with a heat-shrinkable tube, and the entire assembly is heated to effect shrinking. The heating step is followed by removal of the heat-shrinkable tube. In this way the distal tube 30A and the proximal tube 30B are joined together and made mechanically integral (mechanically integrated).

For adhesive bonding, the outer surface of the junction 31 of the proximal tube 30B is coated with an adhesive and is then inserted into the flared end opening 49 of the tube 30A, and the adhesive is cured.

The adhesive may be epoxy adhesive, urethane adhesive, acrylic adhesive, or a mixture thereof. It may be of room-temperature curing type of adhesive, a heat curing type adhesive, or a photopolymerizable type adhesive.

After curing, the junction 31 should have a tensile break strength which is equal to or greater than that of the distal tube 30A.

(e) Outer coating of the medical catheter 10

The medical tube 12 should have its outer surface (particularly that part of the medical tube which comes into contact with blood) coated with an antithrombotic drug, polymeric compound, or silicone oil. The polymeric substance is preferably a hydrophilic or water-soluble one which decreases in frictional coefficient, thereby producing lubricity, upon contact with blood or physiological saline.

The hydrophilic polymeric compound includes, for example, methyl vinyl ether-maleic anhydride copolymer or an ester thereof, polyvinylpyrrolidone, and hydroxypropyl cellulose.

Coating with a hydrophilic polymer compound may be accomplished by dissolving the compound in a solvent such as methyl ethyl ketone, acetone, tetrahydrofuran, dioxane, dimethylformaldehyde, alcohols, and dimethylsulfoxide and applying the solution to the resin tube by dipping, spraying, or the like.

The foregoing step for application is followed by solvent removal by drying or water washing. After solvent removal, the hydrophilic polymeric compound remains in the polymer material constituting the resin tube, thereby imparting lubricity to the surface of the resin tube.

The lubricant applied to the outer surface of the resin tube greatly decreases the area of contact between the outer surface of the resin tube and the inner surface of the body cavity (i.e., instead of the resin tube contacting the body cavity, the lubricant contacts the body cavity) and permits the resin tube (or the medical catheter 10) to slide smoothly in the body cavity.

The lubricating material may be replaced by an antithrombotic drug, such as heparin, polyalkylsulfone, ethyl cellulose, acrylic ester copolymer, methacrylic ester copolymer (e.g., polyhydroxyethyl methacrylate or poly-HEMA), block- or graft-copolymer composed of hydrophobic segments and hydrophilic segments (e.g., HEMA-styrene-HEMA block copolymer, HEMA-MMA [methyl methacrylate] block copolymer, HEMA-LMA [lauryl methacrylate] block copolymer, PVP [polyvinyl pyrrolidone]-MMA block copolymer, HEMA-MMA/AA [acrylic acid] block copolymer), blend polymer thereof incorporated with a polymer having amino groups, and fluororesin.

Preferable among these antithrombotic drugs are HEMA-styrene-HEMA block copolymer, HEMA-MMA block copolymer, and HEMA-MMA/AA block copolymer.

It is desirable to coat the outer surface of the resin tube with any of the antithrombotic drugs (excluding heparin) and heparin sequentially.

The coating of heparin is facilitated if the antithrombotic drug has a hydrophilic group, such as hydroxyl group, amino group, carboxyl group, epoxy group, isocyanate group, thiocyanate group, acid chloride group, aldehyde group, carbon-carbon double bond, or any group replaceable by them.

A blend polymer composed of a hydrophilic resin and a polymer having amino groups is most desirable. A preferred example of the latter is polyamine, particularly PEI (polyethyleneimine).

After an aqueous solution of heparin has been applied to the hydrophilic resin on that surface of the resin tube which comes into contact with blood, heparin is fixed to the hydrophilic resin through covalent bonding with the help of a fixing agent.

Examples of the fixing agent include aldehydes such as glutaraldehyde, terephthalaldehyde, and formaldehyde, diphenylmethane diisocyanate, 2,4-tolylenediiscyanate, carbodiimide-modified diphenylmethane diisocyanate, epichlorohydrin, 1,4-butanediol diglycidyl ether, and polyethylene glycol diglycidyl ether.

The coating of the antithrombotic drug should have an adequate thickness which does not adversely affect the flexibility and outside diameter of the resin tube.

[Examples of typical materials and numerical values]

The following are examples of typical materials and numerical values in the medical catheter (shown in Fig. 1) according to one embodiment.
(1) The distal tube 30A is formed from high-density polyethylene having a flexural modulus of 1600 MPa. It is a resin tube having an inside diameter of 0.87 mm and an outside diameter of 1.03 mm.
(2) The inner tube 32 is formed from PTFE (polytetrafluoroethylene) having a flexural modulus of 550 MPa. It is a resin tube, like the distal tube 30A, which has an inside diameter of 0.87 mm and a wall thickness of 10 $\mu$m. It is used in such a state that it covers the core wire.
(3) The metal mesh layer 34 is formed from flat wires of SUS304. 80 $\mu$m wide and 24 $\mu$m thick.
(4) The coating layer 36 is formed from thermosetting polyimide resin having an elastic modulus of 3100 MPa.
(5) That part (50 cm long) of the outer tube 38 which constitutes the outer tube 38a closer to the junction 31 is formed from high-density polyethylene having a flexural modulus of 1800 MPa. It is a resin tube having an outside diameter of 1.17 mm and an inside diameter of 1.08 mm.
That part of the outer tube 38 which constitutes the outer tube 38b closer to the proximal end is formed from polybutylene terephthalate having a flexural modulus of 2600 MPa. It is a resin tube having an outside diameter of 1.17 mm and an inside diameter of 1.08 mm.

[Example of method for production of medical catheter]

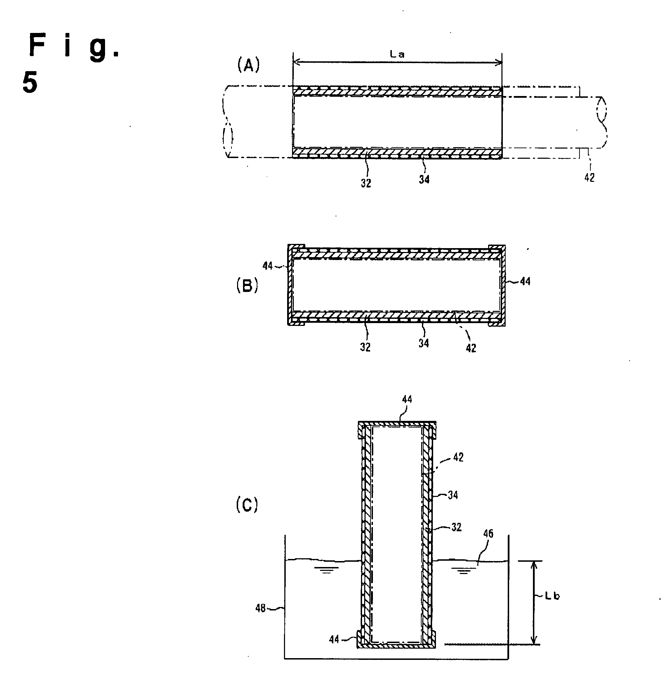

The following is a description of one preferred method by which the medical catheter of the embodiment is produced. The steps of production are schematically shown in Figs. 5 and 6.
First, the inner tube 32 (shown in Fig. 5A) is made ready for use. The inner tube 32 is a PTFE tube covering the core wire 42. It has an inside diameter of 0.87 mm (equal to the outer diameter of the core wire). The inside diameter may range from 0.6 to 0.9 mm.
The inner tube 32 is covered with braided flat metal wires by using a braider having 16 bobbins. The braid has a mesh density of 4 picks per mm Braiding is followed by winding on another bobbin. This braiding step continuously forms the metal mesh layer 34 on the outer surface of the inner tube 32.
The inner tube 32 covered with the metal mesh layer 34 is then cut to a length La (about 130 cm) as generally shown in Fig. 5A.
The inner tube 32 cut to a length (e.g., the length La mentioned above) has both of its ends sealed with a sealant 44 as shown in Fig. 5B. This sealing is intended to fix the metal mesh layer 34 and prevent it from peeling off or separating from the inner tube 32. Sealing protects the core wire 42 from chemicals which would otherwise infiltrate in the subsequent steps.
The resin tube, prepared as described above, is dipped in an etching solution 46 held in a container 48 as shown in Fig. 5C. The depth Lb of dipping is about 200 mm from the distal end. The etching solution is an aqueous solution of ferric chloride. The container of the etching solution is a polypropylene pipe.
After chemical etching (or dipping in the etching solution) for a prescribed period of time, the resin tube is thoroughly washed with tap water. The chemical etching causes the metal mesh layer 34 to become partly thinned so that the metal mesh layer 34 varies in stiffness from one part to another.
The resin tube is coated over its entire length (La) with a thermosetting polyimide resin (of low-temperature curable type) by dipping the resin tube in a solution of the thermosetting polyimide resin. The container of the solution is a polypropylene pipe. After removal from the solution, the coated resin tube is allowed to stand at room temperature and is then heat-cured in a high-temperature oven. This heat curing causes the coating layer 36 to firmly adhere to the inner tube 32 through the metal mesh layer 34 (i.e., through the open spaces of the metal mesh layer) as shown in Fig. 6A. Thus there is obtained a unified composite tube.
After application of the coating layer 36, the resin tube is covered with the outer tube 38a closer to the junction and the outer tube 38b closer to the proximal end. The outer tubes 38a, 38b are formed from different materials. That is, the former outer tube 38a is formed from a soft resin (high-density polyethylene) and the latter is formed from a hard resin (polybutylene terephthalate). Thus, the outer tube 38a closer to the junction is formed from a resin that is softer (not as hard) compared to the resin forming the outer tube 38b closer to the proximal end. This structure makes the outer tube 38b closer to the proximal end stiffer than the outer tube 38a closer to the junction.
As shown in Fig. 6B, the outer tube 38a closer to the junction is positioned such that the coating layer 36 is partly exposed at the junction 31. The exposed part (about 10 mm long) at the distal end of the outer tube 38 serves as the gluing margin.
The resin tube is covered with an easily peelable heat-shrinkable tube, and the covered resin tube is then heated in a specially designed apparatus. Heating causes the heat-shrinkable tube to shrink, and this shrinking causes the coating layer 36 to firmly adhere to the outer tube 38a closer to the junction and the outer tube 38b closer to the proximal end, so that they are mechanically integrated into a single body. After complete cooling, the heat shrinkable tube is removed. The outer tube 38 has an outside diameter of 1.03 mm, which is equal to that of the distal tube 30A.

After removal of the heat shrinkable tube, the core wire 42 in the resin tube is removed by pulling. The opening 49 (at the proximal end) of the distal tube 30A is flared, and the inside of the flare undergoes surface treatment with plasma. The junction 31 of the proximal tube 30B also undergoes surface treatment with plasma. The junction 31 of the proximal tube 30B has its surface coated with an epoxy adhesive, and then it is inserted into the flare of the distal tube 30A as shown in Fig. 6C so that the two tubes are joined together. In this way there is obtained the medical tube 30 composed of the distal tube 30A and the proximal tube 30B which are integrally joined together. The surface treatment increases adhesion strength.

The medical tube 30 is provided with various parts at its distal end and the proximal end according to its use, so that it is made into a medical catheter. For example, the medical tube 12 may be provided with the short tube 14 having a lumen for the guide wire at its distal end 12a and with a connector 16 at its proximal end 12b. In this way, the catheter 10 has useful application for ultrasonic diagnosis.

The etching described above is carried out in such a way that the resin tube is partly dipped in an etching solution for a prescribed period of time. This etching may be repeated while the resin tube is pulled up (i.e., lifted) stepwise. Such stepwise dipping makes the metal mesh layer 34a differ stepwise in thickness in the lengthwise direction of the resin tube. The proximal tube 30B thus prepared has a gradually changing stiffness.

The fabrication steps described above are applicable to a medical tube in which the inner tube 32 has a uniform wall thickness. However, it is also possible to use the described method to produce a medical tube in which the inner tube 32, the coating layer 36, and the outer tube 38 vary in thickness.

The medical tube prepared as described above has a cross-sectional structure as shown in Fig. 3. The distal tube 30A is a resin tube of high-density polyethylene. The junction 31 is composed of a multi-layered structure that includes the inner tube 32 which is a thin tube of PTFE, the metal mesh layer 34a, the coating layer 36 of polyimide resin, and the distal tube 30A of high-density polyethylene resin which is the outer tube of the junction 31.

The segment A is a multilayered structure like the junction 31. However, the segment A has the outer tube 38a closer to the junction on its outermost surface instead of the outer tube 30A. The segment B is a middle part of the proximal tube 30B having the metal mesh. This middle part is covered with the outer tube 38a of high-density polyethylene. The segment C constitutes that part of the resin tube which is closer to the proximal end. The segment C includes the outer tube 38b of polybutylene terephthalate.

The medical tube 12 for medical catheters, prepared as described above, was examined or evaluated for mechanical properties by a three-point bending test as follows. A specimen of the medical tube is placed on supports 20 mm apart and pushed down by a loading nose with a radius of 5 mm at a rate of 10 mm/min. The load at which the specimen achieves elastic recovery is measured. Load measurement is accomplished using an autograph (Model AGS-1kNG from Shimadzu).

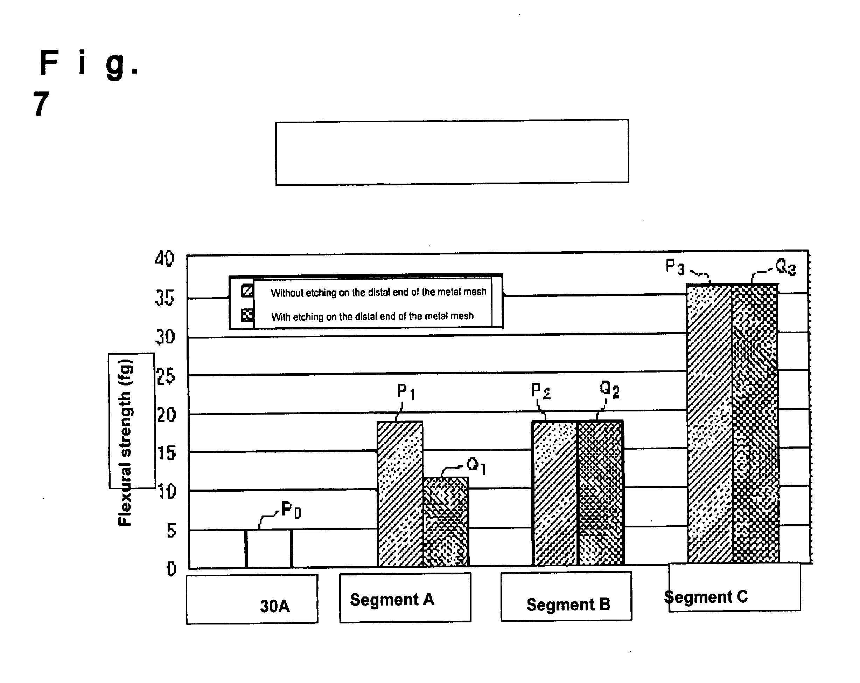

This three-point bending test was performed on both the distal tube 30A and the proximal tube 30B. For comparison purposes, two samples of the proximal tube 30B were evaluated, one in which the distal portion of the metal mesh layer was subjected to etching as described above and another in which the distal portion of the metal mesh layer was not subjected to such etching. The results of the test are shown in Fig. 7.

The specimens were taken from the distal tube 30A at an arbitrary point, the junction 31, and the segments A, B, and C (at a middle point).

It is noted from Fig. 7 that the specimen with the unetched metal mesh layer differs quite significantly in stiffness between the distal tube 30A and the junction 31 (including the segment A) because the proximal tube 30B (closer to the junction 31) is highly stiff. (See bars P0 and P1.) The large difference in stiffness causes kinking.

On the other hand, there is relatively little difference in stiffness between the segments A and B in the specimen with an unetched metal mesh layer. (See bars P1 and P2.) However, there is a difference in stiffness among the segments A, B, and C because the segment C has the outer tube 38b, which has a relatively high stiffness, while the segments A and B include the outer tube 38a which has a relatively low stiffness. (See bars P1, P2 and P3.)

In the case where the metal mesh layer 34a closer to the junction is subjected to etching so that it is made thinner than the metal mesh layer 34b closer to the proximal end, that part of the tube containing the etched metal mesh layer is less stiff than that part of the tube containing the metal mesh layer closer to the proximal end.

Consequently, the difference in stiffness between the distal tube 30A and the junction 31 (including the segment A) is smaller than in the case where no etching is performed. (See bars P0 and Q1.) This leads to freedom from kinking.

In addition, stiffness increases linearly (without abrupt change) in going from the segment A to the segment C. The medical tube with stiffness increasing in such a way is relatively highly resistant to kinking.

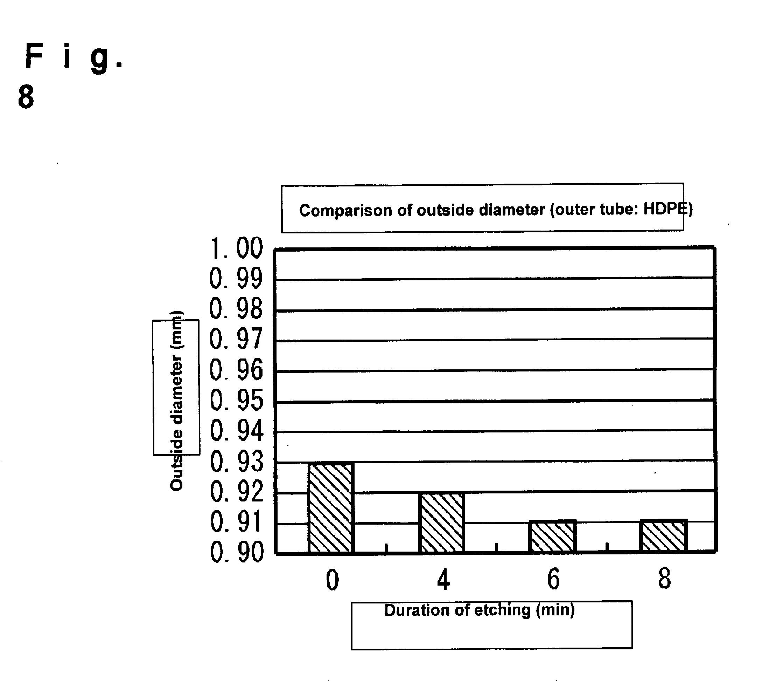

Figs. 8-10 show how the effect of the duration of etching on the outside diameter of the outer tube 38, especially the outer tube 38a closer to the junction, varies depending on the kind of resin constituting the outer tube 38. The duration of etching is 0 (no etching), 4, 6 or 8 minutes. Fig. 8 is a graph showing the change in outside diameter in the case where the outer tube 38a is formed from high-density polyethylene (HDPE). It is noted that the longer the duration of etching, the thinner the metal mesh layer 34a (closer to the junction), and the outer tube 38a (closer to the junction) accordingly has a relatively small outside diameter.

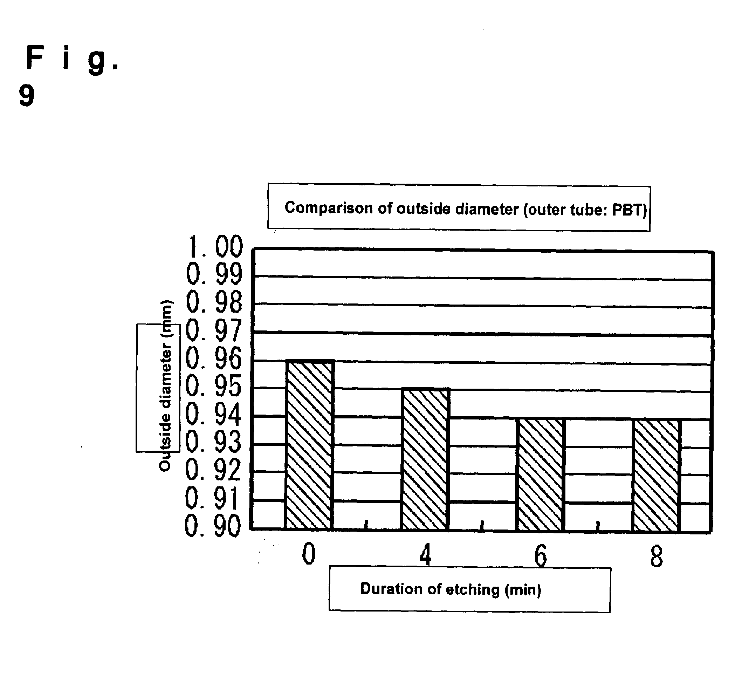

Fig. 9 is a graph showing the change in outside diameter in the case where the outer tube 38a is formed from polybutylene terephthalate (PBT). It is noted that the longer the duration of etching, the smaller the outside diameter of the outer tube 38a (closer to the junction). The rate of decrease is smaller than that shown in Fig. 8.

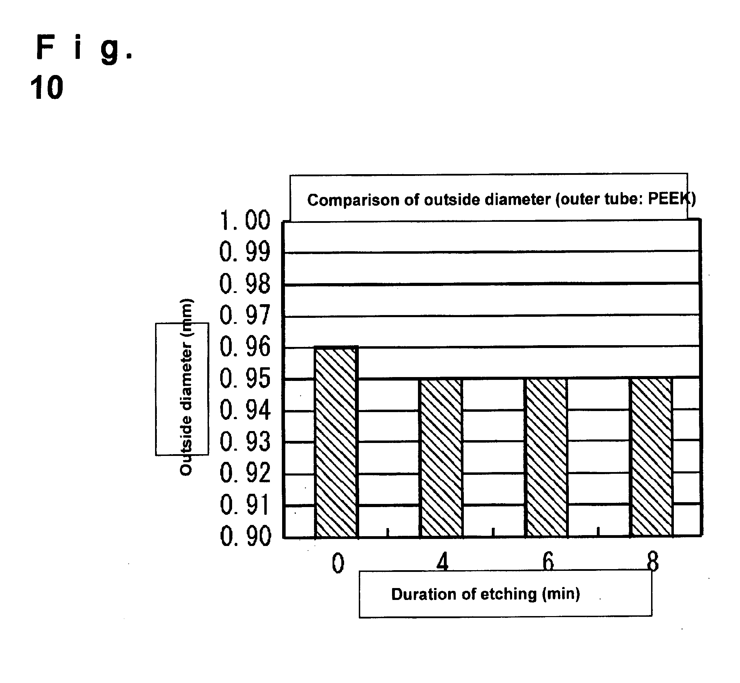

Fig. 10 is a graph showing the change in outside diameter in the case where the outer tube 38a is formed from polyether ether ketone (PEEK). It is also noted that the longer the duration of etching, the smaller the outside diameter of the outer tube 38a (close to the junction). The rate of decrease here is smaller than that shown in Fig. 8.

PEEK has the highest strength, and PBT and HDPE are the second and third. The fact that the outside diameter slightly fluctuates when the duration of etching is 0 minute seems to be attributable to fluctuation in the process.

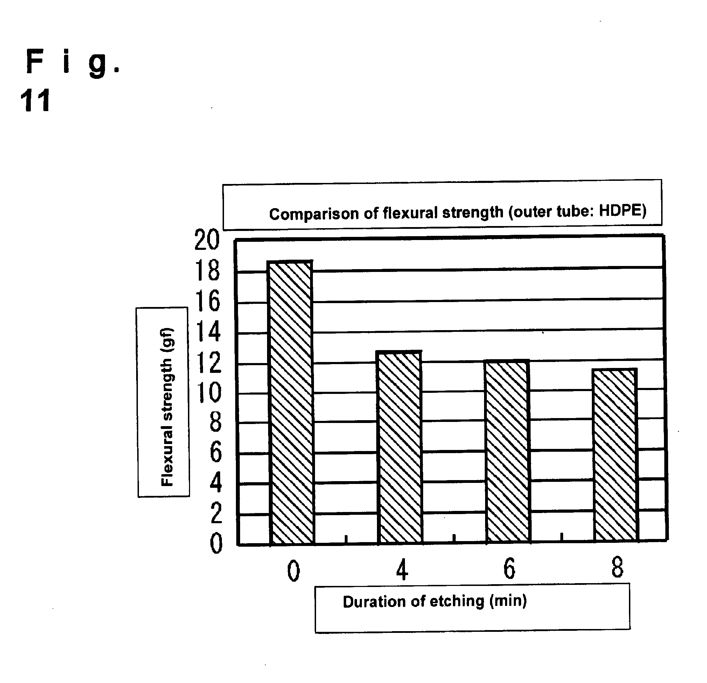

Figs. 11-13 are graphs showing the relationship between the flexural strength and the duration of etching in the case where the outer tube is formed from HDPE, PBT or PEEK, respectively. The duration of etching is the same as mentioned above.

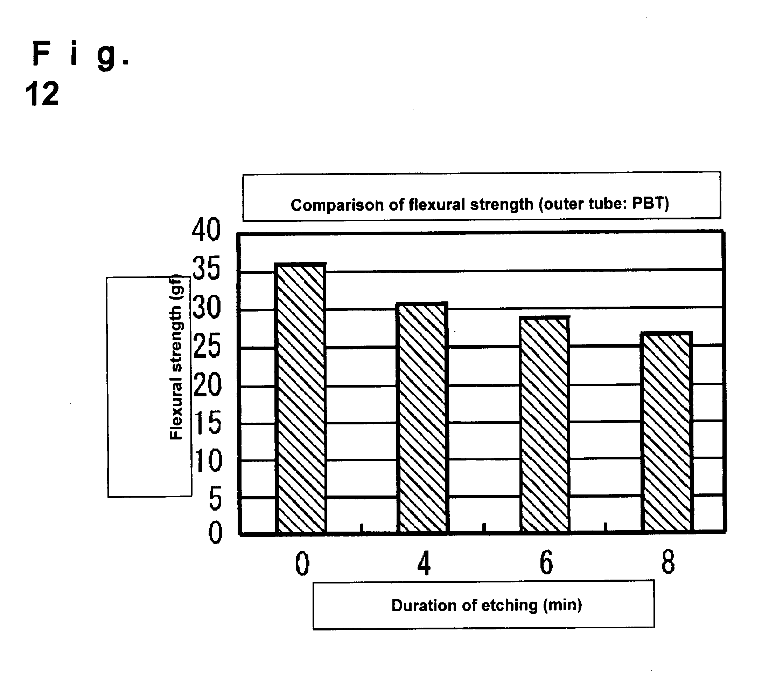

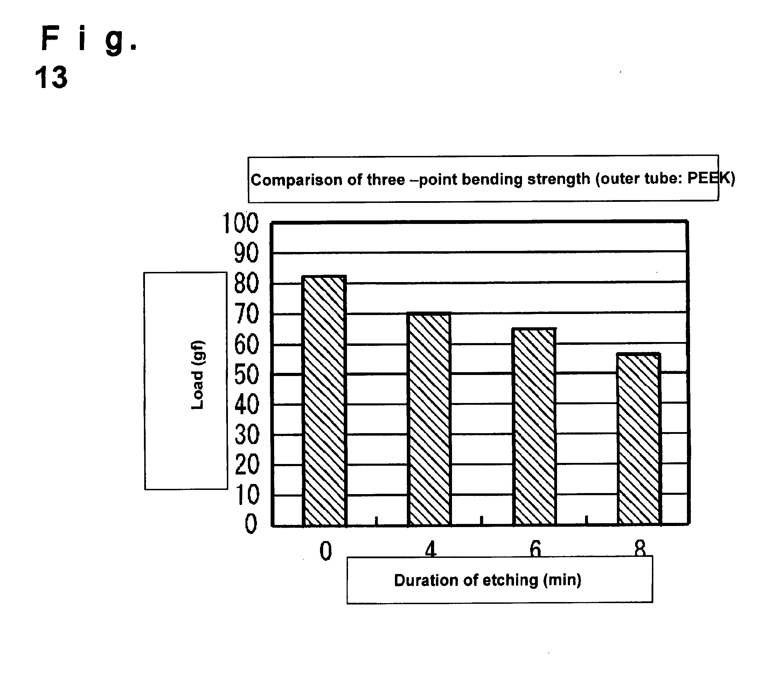

It is noted that the flexural strength depends greatly on the mechanical strength of the outer tube 38a closer to the junction. The outer tube 38a closer to the junction has a small outside diameter and has a low flexural strength accordingly. It is noted that the flexural strength decreases as the outside diameter decreases. For example, in the case shown in Fig. 11, which corresponds to that shown in Fig. 8, the original flexural strength is smaller than the other two cases. Figs. 12 and 13 correspond to Figs. 9 and 10, respectively. There is no significant difference among them in the rate of decrease in flexural strength that depends on the duration of etching.

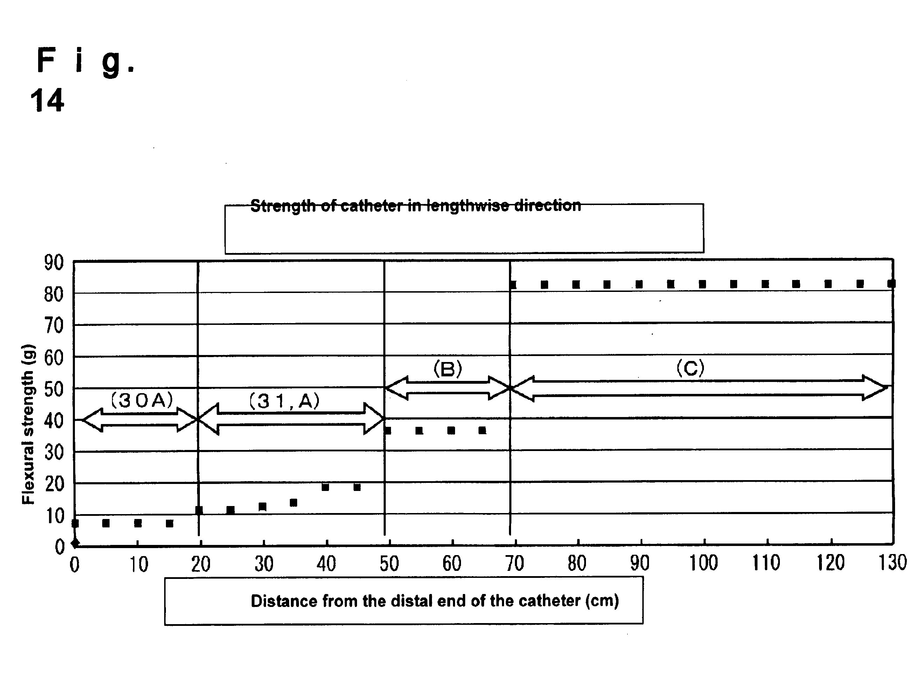

Fig. 14 is a graph showing how the flexural strength changes in the lengthwise direction over the entire length of the medical tube. The medical tube has the etched metal mesh layer 34a close to the junction. In this case, etching is performed on that section of the metal mesh layer corresponding to the segment A (including the junction 31) which ranges from 20 mm to 40 mm. The junction 31 is 15 mm long. The duration of etching increases as the distance increases toward the junction 31. For example, it is 4 minutes in the section of 35 to 40 mm, 6 minutes in the section of 30 to 35 mm, and 8 minutes in the section of 20 to 30 mm.

In the illustrated case, the distal tube 30A and the inner tube 32 are formed from HDPE, the outer tube 38a close to the junction is formed from PBT, and the outer tube 38b close to the proximal end is formed from PEEK.

Fig. 14 is a graph illustrating the flexural strength in more detail than Fig. 7, and it shows how the medical tube changes in flexural strength in its lengthwise direction. The flexural strength is minimum at the distal end 12a close to the short tube 14 provided with a lumen (the catheter 12 is provided with another lumen not connected to the lumen in the short tube 14), and gradually increases in the proximal direction, through the junction 31, the segment A and the segment B. The flexural strength is maximum at the proximal end 12b closer to the connector 16. Thus, the medical tube gradually varies in stiffness in its lengthwise direction, which means that it meets the requirements of the embodiment.

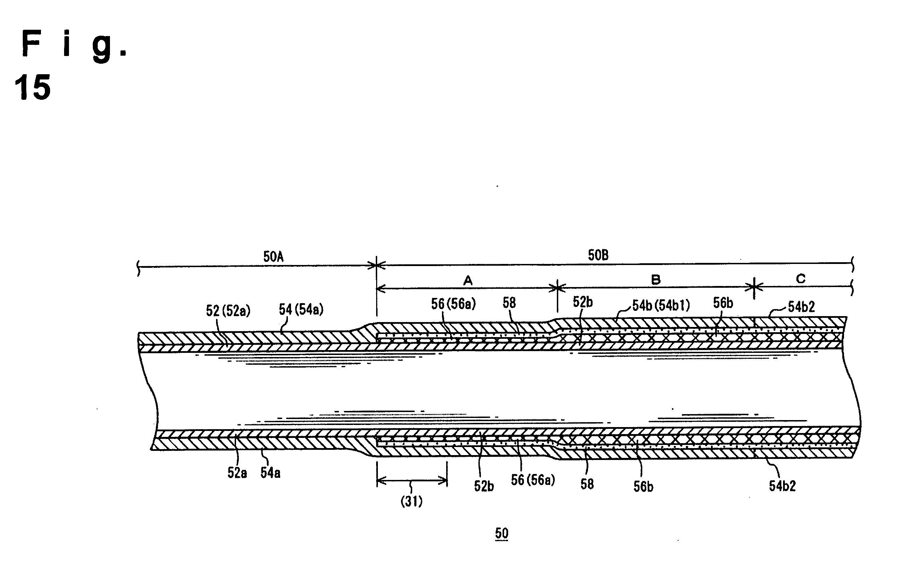

The embodiment described above is concerned with the medical tube that can be applied to a two-piece-type medical catheter. Another embodiment of the medical tube disclosed here as illustrated generally in Fig. 15 is a medical tube that can be applied to a medical catheter of a one-piece type.

The one-piece type medical tube 50 is also composed of the distal tube 50A and the proximal tube 50B. The distal tube 50A has a double-layered structure composed of the inner tube 52a and the outer tube 54a. The proximal tube 50B is composed of the inner tube 52b, the outer tube 54b, the metal layer 56 disposed between the inner tube 52b and the outer tube 54b and the optional coating layer 58 interposed between the two tubes. Thus, the distal end portion of the proximal tube 50B in the region or neighborhood of the distal end of the metal layer 56 is equivalent to the part (or the boundary part) corresponding to the junction 31 shown in Fig. 1. The distal half of the outer tube 54b (which is designated as 54b1) and the proximal half of the outer tube 54b (which is designated as 54b2) are formed from two materials differing in stiffness.

The inner tube 52a and the inner tube 52b constitute an integral body formed from the same resin material. Likewise, the outer tube 54a and the outer tube 54b constitute an integral body formed from the same resin material.

The metal layer 56 is also a metal mesh layer of flat wires. The coating layer 58 fixes the metal mesh layer to the inner tube 52b.

The one-piece type medical tube 50 does not have a junction 31 like the two-piece type medical tube of the first embodiment. The region from the distal end of the metal layer 56 to one portion of the proximal tube 50B corresponds to the boundary (i.e., the boundary between the distal tube 50A and the proximal tube 50B). In addition, the segments A, B and C in the second embodiment shown in Fig. 15 correspond to the segments A, B and C in the first embodiment, except that the segment A is slightly longer by the vanished junction (i.e., the junction from the earlier embodiment that is not present in this embodiment).

The metal mesh layer 56a in the region from the distal end of the metal mesh layer 56 through the segment A is thinner than the metal mesh layer 56b in the segments B and C. Therefore, the medical tube has gradually increasing stiffness from one region to the next in the proximal direction as indicated by the following expression (i.e., as between the distal tube and the segments A, B and C, the distal tube 50A is least stiff and the segment C is most stiff).

$$\text{Distal tube 50A} \leqq \text{segment A} < \text{segment B} < \text{segment C}$$

The resin materials used in this second embodiment are the same as those used in the first embodiment described above. The medical tube in this second embodiment has the same dimensions as that in the first embodiment. Thus, a detailed description of these aspects is not repeated.

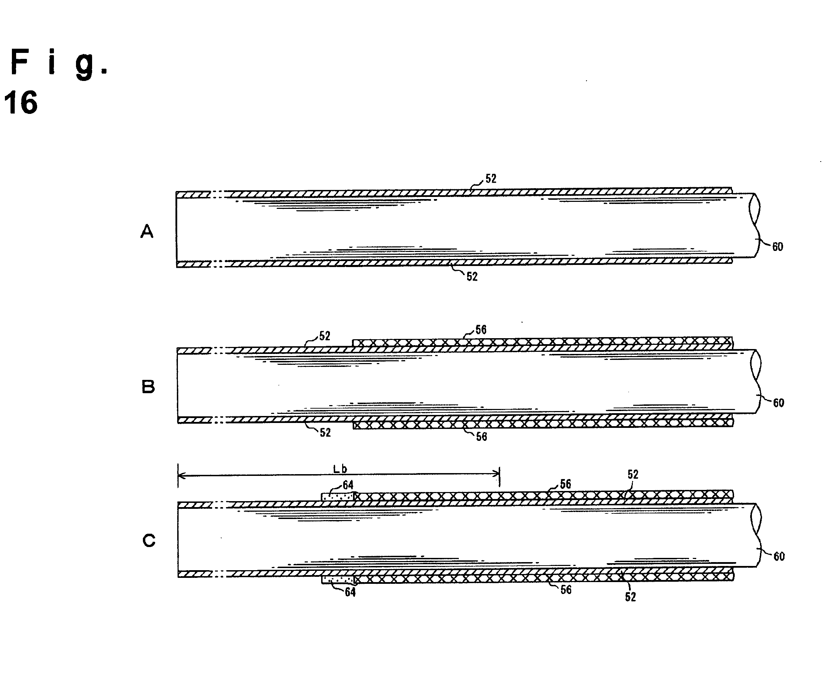

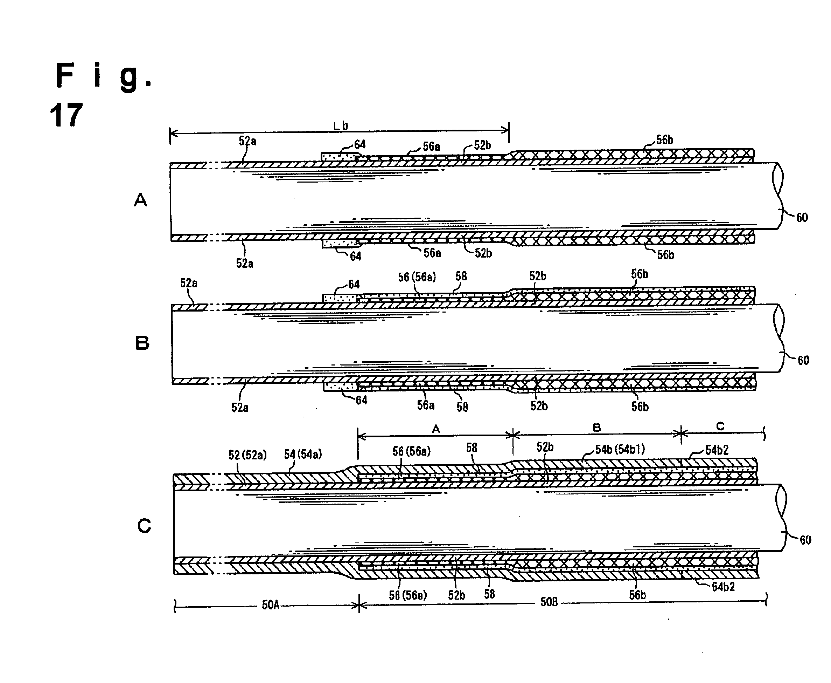

Figs. 16 and 17 show aspects of a method for producing or fabricating the one-piece type medical tube 50. Since the method is similar to that employed for the medical tube of two-piece type, a full detailed description is not repeated. However, a discussion of relevant aspects of the method is set forth below.

First, the inner tube 52 (shown in Fig. 16A), having a length as long as necessary for the medical tube 50, is made. In other words, the inner tube 52 should be as long as the distal tube 50A and the proximal tube 50B combined together. The inner tube 52, which functions as an observation window, is formed from HDPE (high-density polyethylene) on the core wire 60.

That region of the inner tube 52 which corresponds to the proximal tube 50B is covered continuously with the metal mesh layer 56 which is braided with metal flat wires by using a braider. The braiding is generally illustrated in Fig. 16B.

The metal mesh layer or metal braid 56 has its end (closer to the distal tube 50A) sealed with the sealant 64. The sealed region extends a relatively short distance over the inner tube 52 from the end of the metal mesh layer 56 as shown in Fig. 16C. This sealing step fixes the metal mesh layer 56 and prevents it from peeling off or separating outwardly from the inner tube 52.

The resin tube (inner tube with the metal mesh layer) is dipped in an etching solution held in a container similar to that shown in Fig. 5C. The depth Lb of dipping shown in Fig. 16C is longer than the distal tube 50A by about 200 mm.

After chemical etching by dipping in the etching solution, the resin tube is thoroughly washed with tap water. The chemical etching partly thins the metal mesh layer 56, thereby resulting in the metal mesh layer portion 56a which is relatively thinner, and the metal mesh layer 56b which is relatively thicker. In other words, the etched metal mesh layer 56 is composed of two portions differing in thickness, and thus stiffness, as generally illustrated in Fig. 17A.

Thereafter, the resin tube is coated, over at least the entire length of the metal mesh layer 56, with a resin coating material to form the coating layer 58 as shown in Fig. 17B. That part of the resin tube which extends distally beyond the sealant 64 corresponds to the distal tube 50A, and the resin-coated part of the resin tube corresponds to the proximal tube 50B.

The coated resin tube is allowed to stand at room temperature and then is heat-cured in a high-temperature oven. This heat curing causes the coating layer 58 to firmly adhere to the inner tube 52 through the metal mesh layer 56. Thus there is obtained a unified composite tube.

Thereafter, the sealant 64 is removed from the resin tube, and the resin tube is then covered with the outer tube 54 over its entire length as illustrated in Fig. 17C. The outer tube 54 covering the distal tube 50A and the segments A and B of the proximal tube 50B is formed from a first resin material, and the outer tube 54 covering the segment C of the proximal tube 50B is formed from a second and different resin material.

The first resin is a flexible polymeric material highly transparent to ultrasonic waves or visible light so that the outer tube 54 (in combination with the inner tube) functions as an observation window. Examples of such polymeric materials include high-density polyethylene, low-density polyethylene, linear low-density polyethylene, and polyethylene elastomer.

These polymeric materials are highly transparent to both ultrasonic waves and visible light. Therefore, they can be applied to the medical tube (resin tube) designed for diagnosis with ultrasonic waves or visible light. The second resin is a relatively harder resin material. It makes the proximal tube 50B vary in stiffness from one part to the other. That is, the part closer to the proximal end 12b is stiffer than the part closer to the distal end.

The resin tube is covered with an easily peelable heat-shrinkable tube over its entire length, and the covered resin tube is heated in a specially designed apparatus. Heating causes the heat-shrinkable tube to shrink, and this shrinking causes the outer tube 54 to firmly adhere to the inner tube 52, thereby making them mechanically integral, in the distal tube 50A. Similarly, shrinking also causes the outer tube 54 (outer tube portion 54b1 closer to the junction and outer tube portion 54b2 closer to the proximal end) to firmly adhere to the coating layer 58, thereby making them mechanically integral.

The foregoing fabrication method makes integral, by complete adhesion, the end of the metal mesh layer 56 and the inner tube 52 and the outer tube 54 constituting the distal tube 50A. After complete cooling, the core wire 60 and the heat-shrinkable tube are removed from the resin tube. There is thus obtained the medical tube 50 shown in Fig. 15.

Subsequently, the medical tube 50 is provided with various parts at its distal end and the proximal end according to its use, so that it is made into a medical catheter. For example, the medical tube 50 (with a reference number 12 in Fig. 1) may be provided with a short tube 14 (having a lumen) for the guide wire at its distal end 12a and with a connector 16 at its proximal end 12b. In this way there is obtained the catheter 10 for ultrasonic diagnosis.

The medical tube 50 of the one-piece type is simpler from the standpoint of the production process than the medical tube in the first embodiment because it does not require surface treatment for the ends of the distal tube 50A and the proximal tube 50B.

In the first embodiment, the sealant 44 is used to temporarily fix the metal mesh layer 34 (covering the inner tube 32) to the inner tube 32. Similarly, in the second embodiment 2, the sealant 64 is used to temporarily fix the metal mesh layer 56 (covering the inner tube 52b) to the inner tube 52b.

In an illustrative embodiment, instead of using the sealant 44 (or 64), it is possible to fuse the metal layer 34 (or 56). Fusing the metal layer holds individual metal wires together and prevents them from becoming loose. This object is achieved by using a small fusion cutter with a reduced power of gas, laser, or electricity.

The principles, embodiments and modes of operation have been described in the foregoing specification, but the invention which is intended to be protected is not to be construed as limited to the particular embodiments disclosed. The embodiments described herein are to be regarded as illustrative rather than restrictive. Accordingly, it is expressly intended that all such variations, changes and equivalents which fall within the scope of the present invention as defined in the claims, be embraced hereby.

## Claims

1. A medical catheter comprising:

   a distal tube portion (30A, 50A);
   a proximal tube portion (30B, 50B);
   the proximal tube portion (30B, 50B) comprising a metal layer (34, 56) extending longitudinally within the proximal tube portion (30B, 50B); and
   at least a part (34a, ) of the metal layer (34, 56) being subjected to a cross-sectional area reducing treatment resulting in a cross-sectional area of the metal layer (34, 56) varying along a longitudinal extent of the metal layer (34, 56),
   wherein the proximal tube portion (308, 50B) comprises a resin inner layer (32, 52b) and a resin outer layer (38, 54b), the metal layer (34, 56) being positioned between the resin inner layer (32, 52b) and the resin outer layer (38, 54b),
   **characterized in that** the medical catheter (10) further comprises a coating layer (36, 58) between the resin inner layer (32, 52b) and the resin outer layer (38, 54b), the coating layer (36, 58) fixing the metal layer (34, 56) to the resin inner layer (32, 52b), the coating layer (36, 58) possesses a thickness which gradually decreases in a direction from a proximal end (12b) to a distal end (A) of the proximal tube portion (30B, 50B).

2. The medical catheter according to Claim 1, wherein the metal layer (34, 56) extends from a proximal end portion (12b) of the proximal tube portion (30B, 50B) to a distal end portion (A) of the proximal tube portion (303, 50B), the cross-sectional area of the metal layer (34a, 56a) in the distal end portion (A) of the proximal tube portion (30B, 50B) being smaller than the cross-sectional area of the metal layer (34b, 56b) at the proximo end portion (12b) of the proximal tube portion (30B, 50B).

3. The medical catheter according to Claim 1, wherein the metal layer (34, 56) is a metal mesh layer of flat wires.

4. The medical catheter according to Claim 1, wherein the metal layer (34, 56) contacts both the resin inner layer (32, 52b) and the resin outer layer (38, 54b), and wherein the resin inner layer (32, 52b) extends into the distal tube portion (30A, 50A).

5. The medical catheter according to Claim 1, wherein the distal tube (30A) is comprised of a resin tube only.

6. The medical catheter according to Claim 1, wherein the proximal tube portion (50B) and the distal tube portion (50A) both comprise an outer resin layer (54a, 54b) and an inner resin layer (52a, 52b), the outer resin layer (54a) of the distal tube portion (50A) being made of a resin material different from the resin material of which the proximal tube portion (50B) is made.

7. The medical catheter according to Claim 1, wherein the distal tube portion (30A, 50A) is 100-300 mm in length and is comprised of a resin material, and wherein the metal layer (34, 56) does not extend into the distal tube portion (30A, 50A).

8. A method for producing a medical catheter (10) comprised of a distal tube portion (30A, 50A) and a proximal tube portion (30B, 50B), the method comprising:

positioning a metal layer (34, 56) around an inner layer (32, 52b) of the proximal tube portion (30B, 50B) so that the metal layer (34, 56) extends longitudinally along the proximale tube portion (30B, 50B);
forming an outer layer (38, 54b) in covering relation to the metal layer (34, 56) so that the metal layer (34, 56) is positioned between the inner layer (32, 52b) and the outer layer (38, 54b).
treating a distal portion (34a, 56a) of the metal layer (34, 56) to reduce a cross-sectional area of a portion of the longitudinally extending metal layer (34, 56) so that the cross-sectional area of the metal layer (34, 56) varies along a longitudinal extent of the metal layer (34, 56), **characterized in that** the method further comprises applying a coating layer (36, 58) to an exterior surface of the metal, layer (34, 56) prior to forming the outer layer (38, 54b) to fix the metal layer (34, 56) to the inner layer (32, 52b), and treating the coating layer (36, 58) to gradually decrease the thickness in a direction from a proximal end (12b) to a distal end (A) of the proximal tube portion (30B, 50B).

9. The method for producing a medical catheter according to Claim 8, wherein the metal layer (34, 56) is a metal mesh layer comprised of a plurality of wires, and the metal wires are wound around the inner layer (32, 52b),

10. The method for producing a medical catheter according to Claim 8, wherein the metal wires are flat wires.

11. The method for producing a medical catheter according to Claim 8, further comprising joining together the distal tube portion (30A) and the proximal tube portion (30B) after forming an outer layer (38) in covering relation to the metal layer (34) so that the metal layer (34) is positioned between the inner layer (32) and the outer layer (38).

**Patentansprüche**

1. Medizinischer Katheter, der Folgendes umfasst:

einen distalen Tubusabschnitt (30A, 50A),
einen proximalen Tubusabschnitt (30B, 50B),
wobei der proximale Tubusabschnitt (30B, 50B) eine Metallschicht (34, 56) umfasst, die sich in Längsrichtung innerhalb des proximalen Tubusabschnitts (30B, 50B) erstreckt, und
wobei wenigstens ein Teil (34a,) der Metallschicht (34, 56) einer die Querschnittsfläche verringernden Behandlung ausgesetzt wird, die dazu führt, dass sich eine Querschnittsfläche der Metallschicht (34, 56) entlang einer Längsausdehnung der Metallschicht (34, 56) verändert,
wobei der proximale Tubusabschnitt (30B, 50B) eine innere Harzschicht (32, 52b) und eine äußere Harzschicht (38, 54b) umfasst, wobei die Metallschicht (34, 56) zwischen der inneren Harzschicht (32, 52b) und der äußeren Harzschicht (38, 54b) angeordnet ist,
**dadurch gekennzeichnet, dass** der medizinische Katheter (10) ferner eine Überzugsschicht (36, 58) zwischen der inneren Harzschicht (32, 52b) und der äußeren Harzschicht (38, 54b) umfasst, wobei die Überzugsschicht (36, 58) die Metallschicht (34, 56) an der inneren Harzschicht (33, 52b) befestigt, wobei die Überzugsschicht (36, 58) eine Dicke besitzt, die in einer Richtung von einem proximalen Ende (12b) zu einem distalen Ende (A) des proximalen Tubusabschnitts (30B, 50B) allmählich abnimmt.

2. Medizinischer Katheter nach Anspruch 1, wobei sich die Metallschicht (34, 56) von einem proximalen Endabschnitt (12b) des proximalen Tubusabschnitts (30B, 50B) bis zu einem distalen Endabschnitt (A) des proximalen Tubusabschnitts (30B, 50B) erstreckt, wobei die Querschnittsfläche der Metallschicht (34a, 56a) in dem distalen Endabschnitt (A) des proximalen Tubusabschnitts (30B, 50B) kleiner ist als die Querschnittsfläche der Metallschicht (34b, 56b) an dem proximalen Endabschnitt (12b) des proximalen Tubusabschnitts (30B, 50B).

3. Medizinischer Katheter nach Anspruch 1, wobei die Metallschicht (34, 56) eine Metallgitterschicht aus flachen Drähten ist.

4. Medizinischer Katheter nach Anspruch 1, wobei die Metallschicht (34, 56) sowohl die innere Harzschicht (32, 52b) als auch die äußere Harzschicht (38, 54b) berührt und wobei sich die innere Harzschicht (32, 52b) in den distalen Tubusabschnitt (30A, 50A) erstreckt.

5.  Medizinischer Katheter nach Anspruch 1, wobei der distale Tubus (30A) nur aus einem Harztubus besteht.

6.  Medizinischer Katheter nach Anspruch 1, wobei sowohl der proximale Tubusabschnitt (50B) als auch der distale Tubusabschnitt (50A) eine äußere Harzschicht (54a, 54b) und eine innere Harzschicht (52a, 52b) umfassen, wobei die äußere Harzschicht (54a) des distalen Tubusabschnitts (50A) aus einem Harzwerkstoff hergestellt ist, der sich von dem Harzwerkstoff, aus dem der proximale Tubusabschnitt (50B) hergestellt ist, unterscheidet.

7.  Medizinischer Katheter nach Anspruch 1, wobei der distale Tubusabschnitt (30A, 50A) in der Länge 100-300 mm beträgt und aus einem Harzwerkstoff besteht und wobei sich die Metallschicht (34, 56) nicht in den distalen Tubusabschnitt (30A, 50A) hinein erstreckt.

8.  Verfahren zum Herstellen eines medizinischen Katheters (10), der aus einem distalen Tubusabschnitt (30A, 50A) und einem proximalen Tubusabschnitt (30B, 50B) besteht, wobei das Verfahren Folgendes umfasst:

    das Anordnen einer Metallschicht (34, 56) um eine innere Schicht (32, 52b) des proximalen Tubusabschnitts (30B, 50B), so dass sich die Metallschicht (34, 56) in Längsrichtung entlang des proximalen Tubusabschnitts (30B, 50B) erstreckt,
    das Formen einer äußeren Schicht (38, 54b) in Abdeckungsbeziehung zu der Metallschicht (34, 56), so dass die Metallschicht (34, 56) zwischen der inneren Schicht (32, 52b) und der äußeren Schicht (38, 54b) angeordnet ist,
    das Behandeln eines distalen Abschnitts (34a, 56a) der Metallschicht (34, 56), um eine Querschnittsfläche eines Abschnitts der sich in Längsrichtung erstreckenden Metallschicht (34, 56) zu verringern, so dass sich die Querschnittsfläche der Metallschicht (34, 56) entlang einer Längsausdehnung der Metallschicht (34, 56) verändert, **dadurch gekennzeichnet, dass** das Verfahren ferner das Aufbringen einer Überzugsschicht (36, 58) auf eine Außenfläche der Metallschicht (34, 56) vor dem Formen der äußeren Schicht (38, 54b), um die Metallschicht (34, 56) an der inneren Schicht (32, 52b) zu befestigen, und das Behandeln der Überzugsschicht (36, 58), um die Dicke in einer Richtung von einem proximalen Ende (12b) zu einem distalen Ende (A) des proximalen Tubusabschnitts (30B, 50B) allmählich zu vermindern, umfasst.

9.  Verfahren zum Herstellen eines medizinischen Katheters nach Anspruch 8, wobei die Metallschicht (34, 56) eine Metallgitterschicht ist, die aus mehreren Drähten besteht, und die Metalldrähte um die innere Schicht (32, 52b) gewickelt sind.

10. Verfahren zum Herstellen eines medizinischen Katheters nach Anspruch 8, wobei die Metalldrähte flache Drähte sind.

11. Verfahren zum Herstellen eines medizinischen Katheters nach Anspruch 8, das ferner das Verbinden des distalen Tubusabschnitts (30A) und des proximalen Tubusabschnitts (30B) miteinander nach dem Formen einer äußeren Schicht (38) in Abdeckungsbeziehung zu der Metallschicht (34), so dass die Metallschicht (34) zwischen der inneren Schicht (32) und der äußeren Schicht (38) angeordnet ist, umfasst.

**Revendications**

1.  Cathéter médical comprenant :

    une partie tube distal (30A, 50A) ;
    une partie tube proximal (30B, 50B) ;
    la partie tube proximal (30B, 50B) comprenant une couche métallique (34, 56) s'étendant dans le sens longitudinal à l'intérieur de la partie tube proximal (30B, 50B) ; et
    au moins une partie (34a,) de la couche métallique (34, 56) étant soumise à un traitement de réduction de superficie de section transversale qui résulte en une superficie de la section transversale de la couche métallique (34, 56) qui varie sur la longueur de la couche métallique (34, 56),
    dans lequel la partie tube proximal (30B, 50B) comprend une couche intérieure en résine (32, 52b) et une couche extérieure en résine (38, 54b), la couche métallique (34, 56) étant placée entre la couche intérieure en

EP 1 955 724 B1

résine (32, 52b) et la couche extérieure en résine (38, 54b),
**caractérisé en ce que** le cathéter médical (10) comprend en outre une couche de revêtement (36, 58) entre la couche intérieure en résine (32, 52b) et la couche extérieure en résine (38, 54b), la couche de revêtement (36, 58) fixant la couche métallique (34, 56) à la couche intérieure en résine (32, 52b), et la couche de revêtement (36, 58) présente une épaisseur qui diminue progressivement dans une direction allant d'une extrémité proximale (12b) à une extrémité distale (A) de la partie tube proximal (30B, 50B).

2.  Cathéter médical selon la revendication 1, dans lequel la couche métallique (34, 56) s'étend d'une partie d'extrémité proximale (12b) de la partie tube proximal (30B, 50B) à une partie d'extrémité distale (A) de la partie tube proximal (30B, 50B), la superficie de section transversale de la couche métallique (34a, 56a) dans la partie d'extrémité distale (A) de la partie tube proximal (30B, 50B) étant plus petite que la superficie de section transversale de la couche métallique (34b, 56b) au niveau de la partie d'extrémité proximale (12b) de la partie tube proximal (30B, 50B).

3.  Cathéter médical selon la revendication 1, dans lequel la couche métallique (34, 56) est une couche en treillis métallique comprenant des fils plats.

4.  Cathéter médical selon la revendication 1, dans lequel la couche métallique (34, 56) est en contact à la fois avec la couche intérieure en résine (32, 52b) et avec la couche extérieure en résine (38, 54b), et dans lequel la couche intérieure en résine (32, 52b) s'étend dans la partie tube distal (30A, 50A).

5.  Cathéter médical selon la revendication 1, dans lequel le tube distal (30A) est constitué seulement d'un tube en résine.

6.  Cathéter médical selon la revendication 1, dans lequel la partie tube proximal (50B) et la partie tube distal (50A) comprennent toutes deux une couche extérieure en résine (54a, 54b) et une couche intérieure en résine (52a, 52b), la couche extérieure en résine (54a) de la partie tube distal (50A) étant faite d'une résine différente de la résine dont est faite la partie tube proximal (50B).

7.  Cathéter médical selon la revendication 1, dans lequel la partie tube distal (30A, 50A) a une longueur de 100 à 300 mm et est constituée d'une résine, et dans lequel la couche métallique (34, 56) ne s'étend pas dans la partie tube distal (30A, 50A).

8.  Procédé de fabrication d'un cathéter médical (10) comprenant une partie tube distal (30A, 50A) et une partie tube proximal (30B, 50B), le procédé comprenant les étapes consistant à :

    placer une couche métallique (34, 56) autour d'une couche intérieure (32, 52b) de la partie tube proximal (30B, 50B) de sorte que la couche métallique (34, 56) s'étend dans le sens de la longueur de la partie tube proximal (30B, 50B);
    former une couche extérieure (38, 54b) de manière à ce qu'elle recouvre la couche métallique (34, 56) de sorte que la couche métallique (34, 56) est placée entre la couche intérieure (32, 52b) et la couche extérieure (38, 54b) ;
    traiter une partie distale (34a, 56a) de la couche métallique (34, 56) afin de réduire une superficie de section transversale d'une partie de la couche métallique s'étendant dans le sens de la longueur (34, 56) de telle sorte que la superficie de section transversale de la couche métallique (34, 56) varie sur la longueur de la couche métallique (34, 56), **caractérisé en ce que** le procédé comprend en outre l'étape consistant à appliquer une couche de revêtement (36, 58) sur une surface extérieure de la couche métallique (34, 56) avant la formation de la couche extérieure (38, 54b) afin de fixer la couche métallique (34, 56) à la couche intérieure (32, 52b), et à traiter la couche de revêtement (36, 58) pour réduire progressivement l'épaisseur dans une direction allant d'une extrémité proximale (12b) à une extrémité distale (A) de la partie tube proximal (30B, 50B).

9.  Procédé de fabrication d'un cathéter médical selon la revendication 8, dans lequel la couche métallique (34, 56) est une couche en treillis métallique comprenant une pluralité de fils, et les fils métalliques sont enroulés autour de la couche intérieure (32, 52b).

10. Procédé de fabrication d'un cathéter médical selon la revendication 8, dans lequel les fils métalliques sont des fils plats.

11. Procédé de fabrication d'un cathéter médical selon la revendication 8, comprenant en outre l'étape consistant à relier la partie tube distal (30A) et la partie tube proximal (30B) après la formation d'une couche extérieure (38) qui recouvre la couche métallique (34) de telle sorte que la couche métallique (34) est placée entre la couche intérieure (32) et la couche extérieure (38).

# Fig. 1

EP 1 955 724 B1

EP 1 955 724 B1

# F i g. 2

30A

30B

To lumen 14

To connector 16

31

EP 1 955 724 B1

# Fig. 3

(A) Sectional view of the junction 31

(B) Sectional view of the segment A

(C) Sectional view of the segment B

(D) Sectional view of the segment C

EP 1 955 724 B1

Fig. 5

EP 1 955 724 B1

**F i g. 6**

(A)

34 (34a)  32  36  34b  42

(B)

31  39 (38a)

34 (34a)  32  36  34b  38b  42

(C)

30A  49  30B

31

F i g.
8

Comparison of outside diameter (outer tube: HDPE)

Outside diameter (mm)

1.00
0.99
0.98
0.97
0.96
0.95
0.94
0.93
0.92
0.91
0.90

0          4          6          8

Duration of etching (min)

EP 1 955 724 B1

F i g.
9

Comparison of outside diameter (outer tube: PBT)

Outside diameter (mm)

Duration of etching (min)

EP 1 955 724 B1

Comparison of outside diameter (outer tube: PEEK)

Outside diameter (mm)

1.00
0.99
0.98
0.97
0.96
0.95
0.94
0.93
0.92
0.91
0.90

0    4    6    8

Duration of etching (min)

EP 1 955 724 B1

**Fig. 11**

**Comparison of flexural strength (outer tube: HDPE)**

Flexural strength (gf)

20
18
16
14
12
10
8
6
4
2
0

Duration of etching (min)

0    4    6    8

# Fig. 12

EP 1 955 724 B1

Comparison of flexural strength (outer tube: PBT)

Flexural strength (gf)

Duration of etching (min)

Comparison of three –point bending strength (outer tube: PEEK)

Load (gf)

Duration of etching (min)

EP 1 955 724 B1

# F i g.
# 14

EP 1 955 724 B1

Strength of catheter in lengthwise direction

Flexural strength (g)

(30A)  (31, A)  (B)  (C)

Distance from the distal end of the catheter (cm)

EP 1 955 724 B1

# F i g.
## 15

50A | 50B

A | B | C

52 (52a)  54 (54a)   56 (56a)  58   52b   54b (54b1)   56b   54b2

52a  54a

(31)

52b   56 (56a)   58   56b   54b2

50

Fig. 16

EP 1 955 724 B1

Fig. 17

38

**EP 1 955 724 B1**

**REFERENCES CITED IN THE DESCRIPTION**

**Patent documents cited in the description**

- US 5533987 A
- JP 2001178826 A
- WO 2005018727 A
- WO 0013733 A
- EP 0841072 A